(19) Europäisches Patentamt
European Patent Office
Office européen des brevets

(11) Numéro de publication: 0 341 154
A2

(12) # DEMANDE DE BREVET EUROPEEN

(21) Numéro de dépôt: 89401251.7

(22) Date de dépôt: 03.05.89

(51) Int. Cl.[4]: C 07 H 3/02
C 07 H 7/02, C 07 H 13/08,
C 07 H 15/18, C 07 H 15/252,
A 61 K 31/70, C 07 D 317/20,
C 07 D 317/22, C 07 D 317/24

(30) Priorité: 04.05.88 FR 8805969

(43) Date de publication de la demande:
08.11.89 Bulletin 89/45

(84) Etats contractants désignés:
AT BE CH DE ES FR GB GR IT LI LU NL SE

(71) Demandeur: LABORATOIRES HOECHST S.A.
TOUR ROUSSEL HOECHST, 1 Terrasse Bellini
F-92800 Puteaux (FR)

BEHRINGWERKE Aktiengesellschaft
Postfach 1140
D-3550 Marburg 1 (DE)

(72) Inventeur: Gesson, Jean-Pierre
"La Germonière" Montamise
F-86360 Chasseneuil-du-Poitou (FR)

Jacquesy, Jean-Claude
46 rue du Planty
F-86180 Buxerolles (FR)

Petit, Patricia
Place de Rochemaux Bât. 6, Appt. 611
F-86000 Poitiers (FR)

Kraemer, Hans-Peter
Birkenweg 16
D-3550 Marburg (DE)

Mondon, Martine
9 rue du Pontreau Appt 529
F-86000 Poitiers (FR)

(74) Mandataire: Brycman, Jean et al
CABINET ORES 6, Avenue de Messine
F-75008 Paris (FR)

(54) **Nouveaux analogues du L-fucose, leur procédé de préparation, application de ces analogues à la préparation de nouveaux glycals, anthracyclines obtenues à l'aide de ces glycals et utilisation desdites anthracyclines en tant que médicaments.**

(57) La présente invention est relative à de nouveaux analogues du L-Fucose, à leur procédé de préparation, à l'application de ces analogues à la préparation de nouveaux glycals, aux anthracyclines obtenues à l'aide de ces glycals et à l'utilisation desdites anthracyclines en tant que médicaments.

Les glycals sont obtenus à partir du L-Fucose et/ou de ses analogues de Formule 1' :

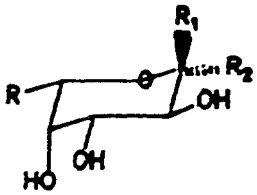

(1')

et répondent à la Formule 2, ci-après :

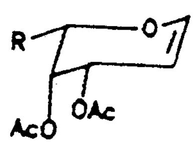

(2)

dans laquelle :

**R** représente un groupe alkyle éventuellement substitué, linéaire ou ramifié ou un groupe aryle éventuellement substitué, à l'exception d'un groupe $CH_2OH$ ou d'un groupe $CH_2OR_3$, $R_3$ ayant la même signification que R.

EP 0 341 154 A2

**Description**

**NOUVEAUX ANALOGUES DU L-FUCOSE, LEUR PROCEDE DE PREPARATION, APPLICATION DE CES ANALOGUES A LA PREPARATION DE NOUVEAUX GLYCALS, ANTHRACYCLINES OBTENUES A L'AIDE DE CES GLYCALS ET UTILISATION DESDITES ANTHRACYCLINES EN TANT QUE MEDICAMENTS.**

La présente invention est relative à de nouveaux analogues du L-Fucose, à leur procédé de préparation, à l'application de ces analogues à la préparation de nouveaux glycals, aux anthracyclines obtenues à l'aide de ces glycals et à l'utilisation desdites anthracyclines en tant que médicaments.

Les anthracyclines constituent une famille importante d'agents anticancéreux. Leur inconvénient majeur est leur cardiotoxicité ; l'atteinte cardiaque est fonction de la dose totale administrée ; toutefois, celle-ci peut varier selon l'anthracycline.

Le L-Fucose présente un grand intérêt biologique puisqu'il est un des constituants de plusieurs antigènes de groupes sanguins et que plusieurs glycosides, dérivés du désoxy-2-L-Fucose, aisément obtenu à partir du L-Fucose, présentent des activités antibiotiques et antitumorales en série anthracycline.

En particulier, le glycoside de Formule 3a (D. HORTON et al., Carbohydr. Res., 1977, 57, C36)

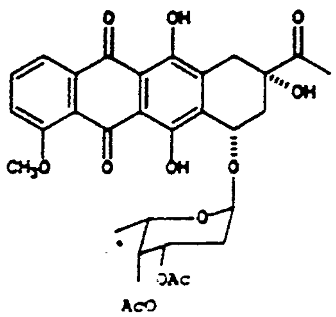

(3a)

présente une activité antitumorale élevée *in vivo*, similaire à celle de la daunorubicine de formule 4a (D. HORTON et al., J. Med. Chem., 1976,22,406).

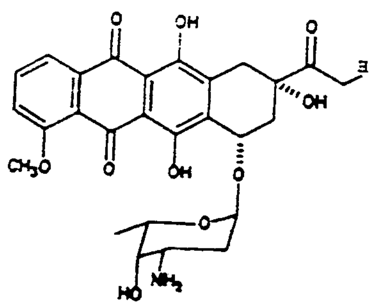

(4a)

Le désoxy-2-L-Fucose est aussi le constituant central de nombreux trisaccharides en série anthracycline, comme l'aclacinomycine A de Formule 5 :

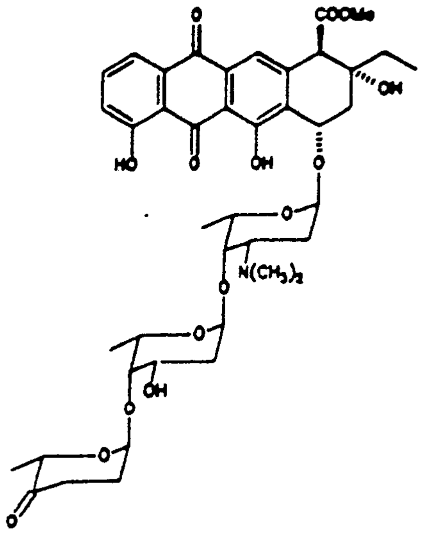

(5)

qui présentent également une activité antitumorale remarquable.

De nombreux efforts sont poursuivis dans ce domaine, dans le but d'améliorer l'index thérapeutique des anthracyclines, mais aussi pour trouver des composés actifs sur des souches de cellules cancéreuses résistantes aux anthracyclines utilisées actuellement en clinique. Une des modifications les plus prometteuses paraît être celle de la partie glycosidique qui est impliquée dans toutes les étapes du mode d'action de ces composés (transport, accumulations intracellulaires, complexation avec l'ADN ...).

C'est pourquoi, la Demanderesse, poursuivant l'étude entreprise concernant la synthèse de nouveaux glycosides de grande valeur thérapeutique (cf notamment Brevets français n° 84 03634 et n° 85 10063), a mis au point un nouveau et tant économiquement qu'industriellement très intéressant procédé de synthèse du L-Fucose et d'analogues de celui-ci, indispensables à la préparation de nouvelles anthracyclines.

Malheureusement, le L-Fucose (ou désoxy-6-L- galactose) de Formule 1 :

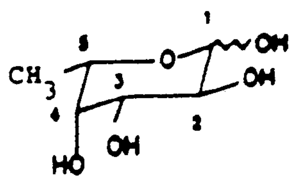

(1)

est un sucre obtenu difficilement par extraction d'espèces de Fucus variées et est donc d'un prix de revient élevé. Plusieurs synthèses totales ont déjà été proposées , mais elles se caractérisent par un nombre d'étapes élevé ou par la nécessité d'une étape de séparation peu adaptée à la préparation de quantités importantes (DEFAYE et al., Carbohydr. Res., 126, 165, 1984).

La présente invention s'est en conséquence donnée pour but de fournir de nouvelles anthracyclines qui répondent mieux aux nécessités de la pratique que les anthracyclines de l'Art antérieur, leur procédé de préparation ainsi que les sucres et les glycals servant à leur préparation.

La présente invention a pour objet des analogues du L-Fucose, correspondant à la formule générale 1′

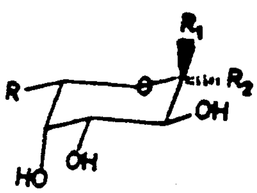

(1′)

dans laquelle :

**R** représente un groupe alkyle éventuellement substitué, linéaire ou ramifié ou un groupe aryle éventuellement substitué, à l'exception d'un groupe méthyle, d'un groupe $CH_2OH$ ou d'un groupe $CH_2OR_3$, $R_3$ ayant la même signification que R ;

**R et $R_2$** sont toujours différents et représentent un atome d'hydrogène ou un groupe hydroxyle, étant donné que lorsque $R_1$ est un atome d'hydrogène et $R_2$ est un groupe OH, on obtient un stéréoisomère β et lorsque $R_1$ est un groupe hydroxyle et $R_2$ est un atome d'hydrogène, on obtient un stéréoisomère α.

La présente invention a également pour objet un procédé de préparation du L-Fucose de Formule 1 et/ou de ses analogues de Formule 1′, à partir de D-mannose, caractérisé :

a. en ce qu'on transforme le D-mannose de Formule 6 en diacétone mannose de Formule 7,

b. en ce qu'on condense un dérivé organométallique de formule R-Y, dans laquelle :
**R** est un groupe alkyle linéaire ou ramifié insaturé ou non, éventuellement substitué ou un groupe aryle substitué ou non, y compris un groupe méthyle et
**Y** du magnésium ou du lithium
avec le diacétone mannose de Formule 7, ladite condensation fournissant :
- le diol de Formule 8, dans le cas où R représente un groupe méthyle, et

c. en ce qu'on protège les fonctions alcool dudit diol de Formule 8 par un traitement par un composé de formule R′-X, dans laquelle :
**R′** est un groupe benzyle éventuellement substitué et
**X** un halogène
pour obtenir le composé de Formule 9,

d. en ce qu'on hydrolyse, au cours d'une quatrième étape, le composé de Formule 9 pour donner le produit de Formule 10,

e. en ce qu'on scinde le produit de Formule 10, au niveau du glycol en bout de chaîne, au cours d'une cinquième étape pour obtenir un mélange de deux anomères α et β de Formule 11,

f. en ce que l'on transforme le mélange obtenu au cours de l'étape e. en produit recherché (1), au cours d'une sixième étape, par hydrogènolyse,
selon le schéma I suivant :

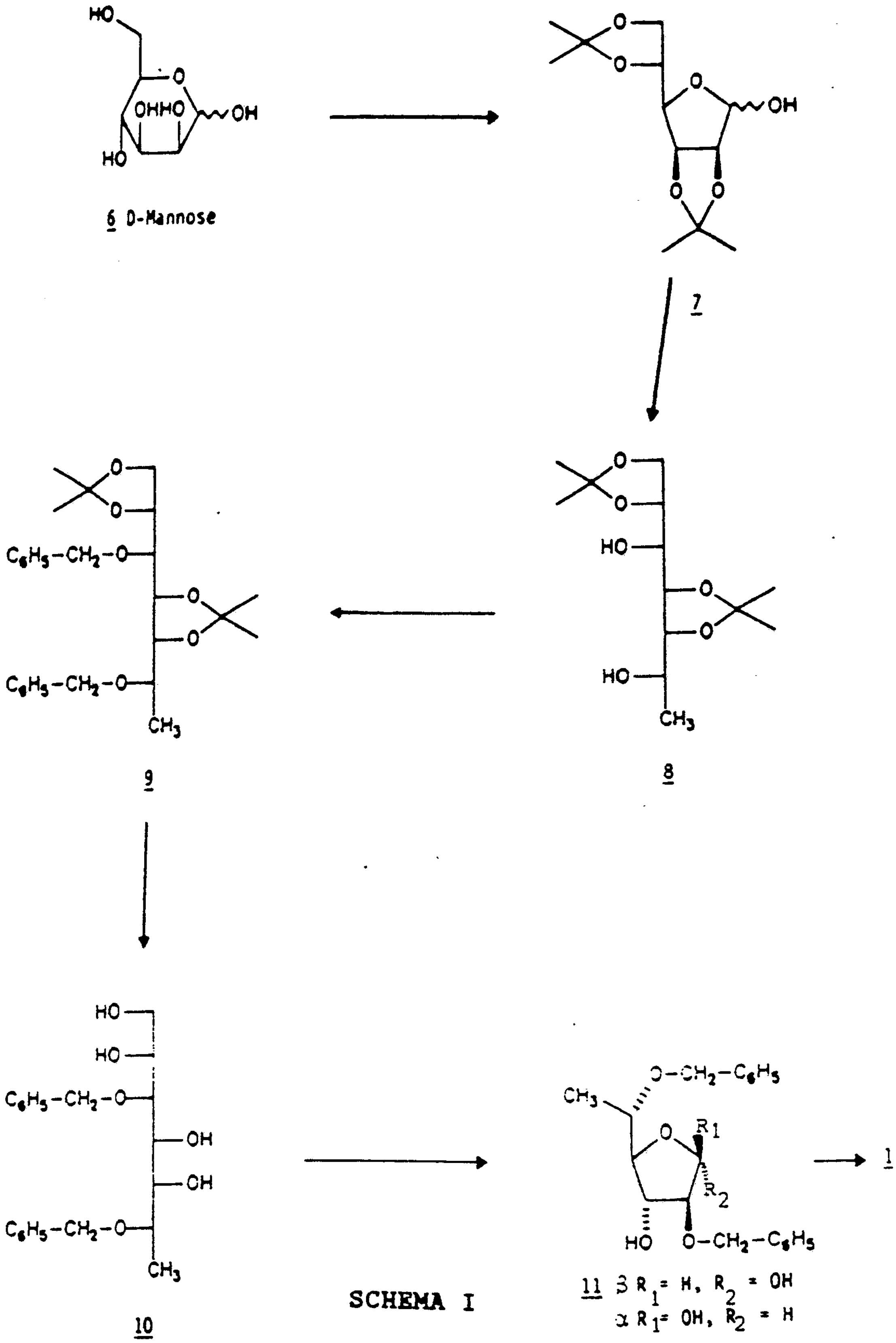

6 D-Mannose

7

8

9

10

$\underline{11}$ β $R_1$ = H, $R_2$ = OH
α $R_1$ = OH, $R_2$ = H

→ 1

SCHEMA I

- un mélange de deux diols 12 et 13, dans le cas où R représente un groupe alkyle éventuellement substitué, linéaire ou ramifié ou un groupe aryle éventuellement substitué, à l'exception d'un groupe méthyle, d'un groupe $CH_2OH$ ou d'un groupe $CH_2OR_3$, $R_3$ ayant la même signification que R, et

c. en ce qu'on protège les fonctions alcool dudit mélange de diols 12 et 13 par un traitement par un composé de formule R'-X, dans laquelle :

**R'** est un groupe benzyle éventuellement substitué et

**X** un halogène
pour obtenir les composés de Formules 14 et 15,

d. en ce qu'on hydrolyse, au cours d'une quatrième étape, le composé 14 pour donner le produit de Formule 16,

e. en ce qu'on scinde le produit de Formule 16, au niveau du glycol en bout de chaîne, au cours d'une cinquième étape pour obtenir un mélange de deux anomères α et β de Formule 17,

f. en ce que l'on transforme le mélange obtenu au cours de l'étape e. en produit recherché (1′), au cours d'une sixième étape, par hydrolyse,
selon le schéma II suivant :

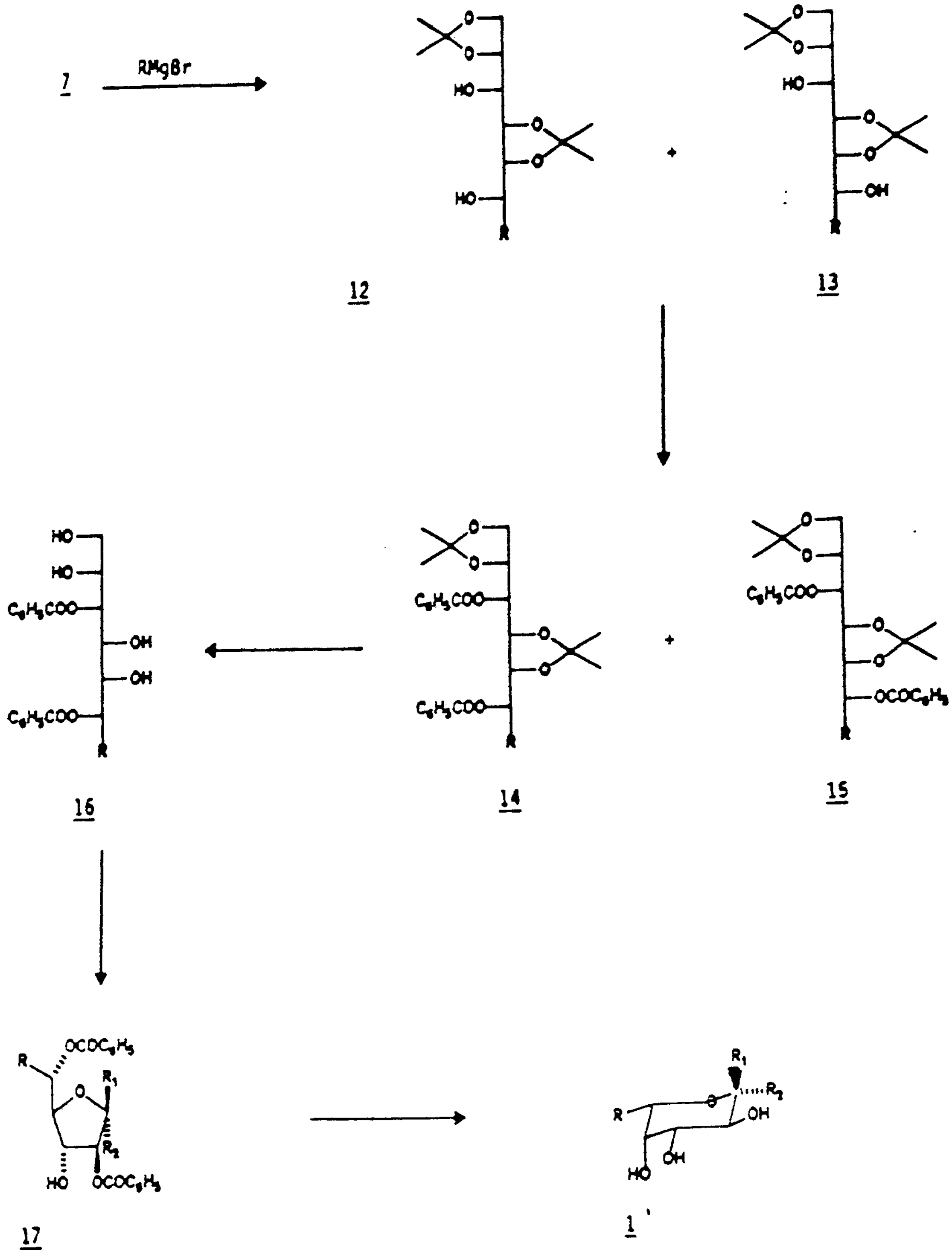


7 12 13 14 15 16 17 1'

SCHEMA II

La présente invention a également pour objet des glycals obtenus à partir du L-Fucose et/ou de ses analogues selon l'invention, de Formule 2 :

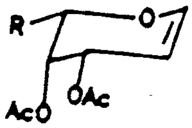

(2)

dans laquelle :
**R** représente un groupe alkyle éventuellement substitué, linéaire ou ramifié ou un groupe aryle éventuellement substitué, à l'exception d'un groupe $CH_2OH$, d'un groupe $CH_2OR_3$, $R_3$ ayant la même signification que R.

Les glycals de Formule 2 sont obtenus de manière connue selon la méthode de B. Iselin et T. Reichstein (Helv. Chim. Acta, 1944, 27, 1200) qui comprend les étapes suivantes :

Première étape :
peracétylation d'un mélange d'ancmères α et β de Formule 1′ pour obtenir un dérivé sous forme d'isomères α et β de Formule 18 :

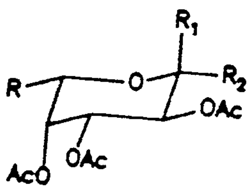

(18)

dans laquelle R a la même signification que précédemment ;
$R_1$ est un hydrogène et $R_2$ un groupe acétoxy ;
l'isomère 18 β peut être séparé par précipitation sélective.

Deuxième étape :
traitement du mélange 18 α et β par de l'acide bromhydrique, pour obtenir un dérivé bromé intermédiaire qui, soumis à l'action du zinc fournit un glycal de Formule 2, selon le schéma III suivant :

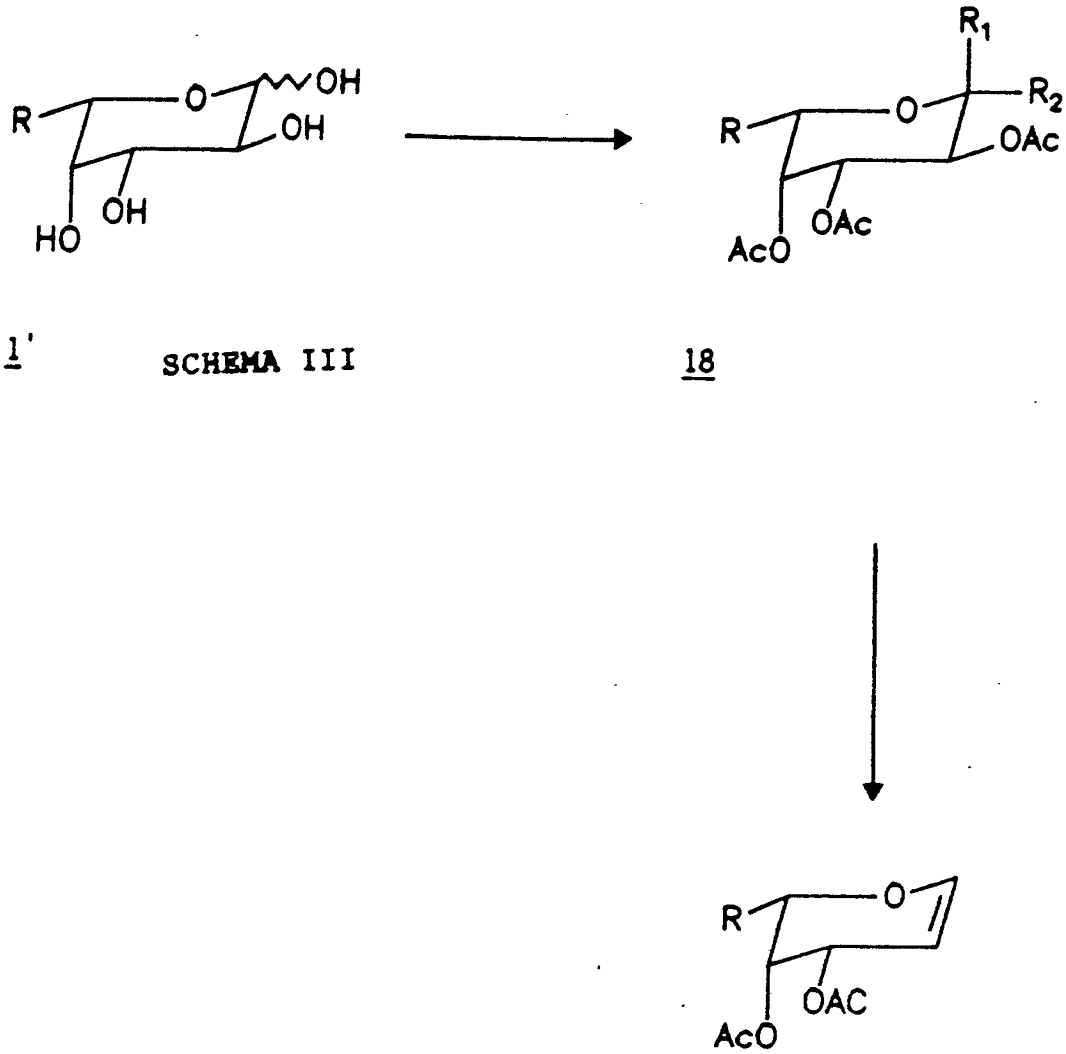

1'    SCHEMA III    18

2

La présente invention a, en outre, pour objet un procédé de préparation de nouvelles anthracyclines à partir d'un glycal selon l'invention, caractérisé en ce que :

a. ledit glycal de Formule 2 est transformé en dérivé halogéné par addition d'un composé de Formule HX anhydre, dans laquelle X est un halogène, pour obtenir un composé de Formule 19, selon le schéma IV.

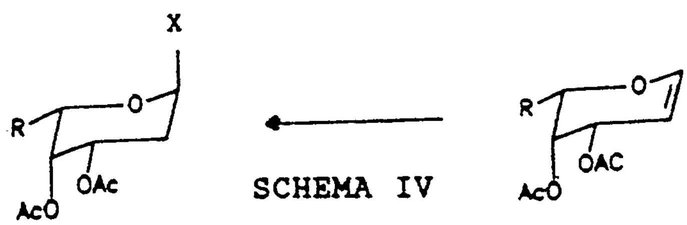

19    SCHEMA IV    2

b. puis ledit composé de Formule 19 est couplé à une anthracyclinone appropriée, par une réaction de type Koenigs-Knorr.

Selon un mode de mise en oeuvre dudit procédé, ledit composé 19 est couplé avec la daunomycinone et fournit un produit de Formule 20′ :

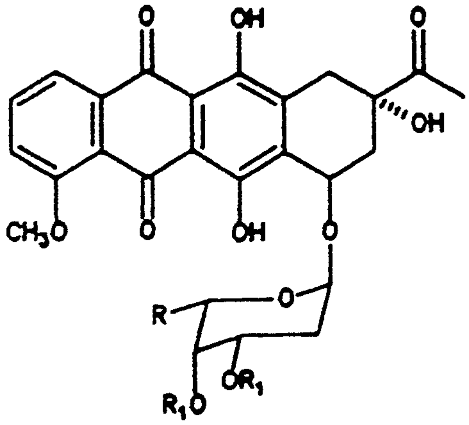

(20')

dans laquelle :
**R** est tel que défini ci-dessus ;
**$R_1$** représente un groupe acétyle ou un atome d'hydrogène.

Selon un autre mode de mise en oeuvre dudit procédé, ledit composé 19 est couplé avec la β rhodomycinone et fournit un produit de Formule 21'.

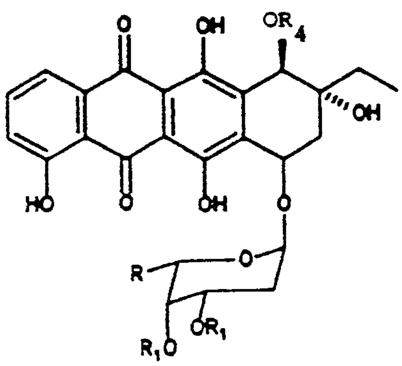

(21')

dans lequel,
**R** est tel que défini ci-dessus ;
**$R_1$** représente un groupe acétyle ou un atome d'hydrogène ;
**$R_4$** représente un atome d'hydrogène ou le radical de Formule 19',

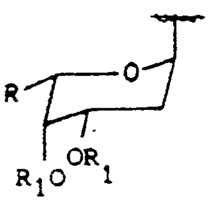

(19')

dans lequel R et $R_1$ ont les mêmes significations que précédemment.

Les glycosides obtenus sont purifiés par chromatographie.

Outre les dispositions qui précèdent, l'invention comprend encore d'autres dispositions qui ressortiront de la description qui va suivre.

L'invention vise plus particulièrement les nouveaux analogues du L-Fucose et de nouveaux glycals obtenus à partir desdits analogues ainsi que de nouvelles anthracyclines, et leurs procédés de préparation, ainsi que des compositions, en particulier des compositions thérapeutiques, dans lesquelles entrent lesdits dérivés, pour leur utilisation en chimiothérapie anti-cancéreuse.

Ces nouvelles anthracyclines ont des propriétés cytotoxiques et antitumorales remarquables, comme précisé dans les tests pharmacologiques ci-après.

Parmi les différentes anthracyclines conformes à la présente invention, présentant des résultats pharmacologiques très intéressants, il y a lieu de citer notamment :

. la Di-O-(di-O-acétyl-3',4' didésoxy-2',6'-α-L-thréo-hexopyrannosyl) - 7,10 β-Rhodomycinone de Formule 22a ;

. la O-(di-O-acétyl-3',4' didésoxy-2',6'-α-L-thréo-hexopyrannosyl)-7 β-Rhodomycinone de Formule 21a ;

- la Di-O-(di-O-acétyl-3',4'tridésoxy-2',6',7'-α-L-thréo heptopyrannosyl)-7,10 β-Rhodomycinone de Formule 22b ;

- la O-(di-O-acétyl-3′,4′ tridésoxy-2′,6′,7′-α-L-thréo-heptopyrannosyl)-7 β-Rhodomycinone de Formule 21b ;
- la di-O-(di-O-acétyl-3′,4′ hexadésoxy-2′,6′,7′,8′,9′, 10′-α-L-thréo décapyrannosyl)-7,10 β-Rhodomycinone de Formule 22 c;
. la O-(di-O-acétyl-3′,4′hexadésoxy-2′,6′,7′,8′,9′,10′-α-L-thréo décapyrannosyl)-7 β-Rhodomycinone de Formule 21c ;
. l'acétoxy-3′ O-acétyl-4′ désamino-3′ méthyl-6′ daunorubicine de Formule 20b ;
. l'acétoxy-3′ O-acétyl-4′ désamino-3′ propyl-6′ daunorubicine de Formule 20c.

L'invention sera mieux comprise à l'aide du complément de description qui va suivre, qui se réfère à des exemples de mise en oeuvre de procédé, objet de la présente invention ainsi qu'à des exemples mettant en évidence les propriétés thérapeutiques desdits glycosides.

En I.R., les bandes sont données en $cm^{-1}$.

En RMN, les déplacements chimiques sont donnés en ppm par rapport au tétraméthylsilane (TMS) utilisé comme référence.

**EXEMPLE 1 : SYNTHESE DU L-FUCOSE :**

Cette synthèse nécessite seulement six étapes, à partir d'un sucre très accessible, le D-mannose de Formule 6.

. Première étape :

Le D-mannose 6 est transformé quantitativement en diacétone mannose 7 selon la méthode décrite par J.B. Lee et T.T. NOLAN (Tetrahedron, 1967, 23, 2789).

. Deuxième étape :

On additionne lentement une solution 1,6 M de $CH_3Li$ dans l'hexane (45 ml, 0,072 mole) à une solution de diacétone mannose 7 (3g, 0,0115 mole) dans le tétrahydrofuranne (THF) anhydre (45 ml), maintenue à -40°C.

Après 1 h. à cette température, le mélange réactionnel est agité une nuit à 0°C, puis jeté sur un mélange eau-glace-$NH_4Cl$. Après extraction usuelle au dichlorométhane, on obtient l'alcool 8 (diol) (2,95g, rendement = 95 %), suffisamment pur pour être utilisé dans l'étape suivante.

L'utilisation du THF, dans la mise en oeuvre décrite n'est pas limitative, d'autres solvants anhydres peuvent être utilisés, tels que alcanes, éthers, et ce à une température appropriée.

**. Di-O-isopropylidène-1,2,4 5 hexahydroxy-1, 2R, 3R, 4R, 5R, 6S heptane de Formule 8 :**
**Huile**
$(\alpha)_D$ = -9,7° (c = 1,84, $CHCl_3$)
I.R ($CH_2Cl_2$) : 3690, 3420, 2995, 2945, 1450, 1380, 1242, 1212, 1150, 1070, 1010, 960, 930, 880, 848.
RMN ($CDCl_3$) : 1,29 (d., J = 7 Hz, 3H) ; 1,37 (s, 3H) ; 1,41 (s, 6H) ; 1,55 (s, 3H) ; 3,60 (d, J = 6Hz, 1H) ; 4,0-4,2 (m, complexe, 5H) ; 4,41 (d., J = 6Hz, 1H).

**Troisième étape :**

L'alcool précédent 8 (2g, 0,0072 mole) est dissout dans du tétrahydrofuranne (THF) anhydre (20 ml) contenant de l'hydrure de sodium à 50 % dans l'huile (1g, 0,0208 mole). On additionne alors le bromure de benzyle (1,95 ml, 2,8 g) puis du HMPT (1 ml). Le mélange réactionnel est porté une nuit à reflux puis est hydrolysé dans l'eau glacée. Après acidification avec de l'acide chlorhydrique 0,1N, une extraction usuelle fournit une huile qui est filtrée sur une petite colonne de silice (éluant, éther de pétrole) pour éliminer l'huile contenant initialement l'hydrure et le bromure de benzyle n'ayant pas réagi. Une élution à l'éther fournit alors le dérivé 9 recherché (2,47 g - rendement = 75 %).

. Di-O-benzyl-3,6 di-O-isopropylidène-1,2,4-5 hexahydroxy-1,2R,3R,4R,6S heptane de Formule 9 :
Huile :
$(\alpha)_D$ = -3° (C = 1, $CHCl_3$).
I.R. ($CH_2Cl_2$) : 2990, 2940, 1490, 1445, 1370, 1230, 1210, 1135, 1060.
RMN ($CDCl_3$) : 1,18 (d., J = 7 Hz, 3 H) ; 1,32 (s, 3H) ; 1,38 (s, 6H) ; 1,56 (s, 3H) ; 3,75 - 4,25 (m, complexe, 7H) ; 4,54 (d, J = 12 Hz, 1H) ; 4,63 (2d., J = 12 Hz, 2H) ; 4,78 (d., J = 12 Hz, 1H), 7,1-7,4 (m, 10H).

**. Quatrième étape :**

Une solution du diacétonide 9 (4,17g, 0,009 mole) dans l'acide acétique (184 ml) et l'eau (45 ml) est agitée à 60°C pendant une heure. Après évaporation sous vide du solvant, du toluène (50 ml) est additionné puis évaporé de nouveau. Le résidu cristallisé est mis en suspension dans l'éther de pétrole, puis filtré. On obtient ainsi le tétrol recherché 10 (3,19 g, rendement = 92 %).

L'utilisation d'un mélange acide acétique - eau pour effectuer l'hydrolyse, dans la mise en oeuvre décrite, n'est pas limitative, d'autres mélanges peuvent être utilisés tels que $HCl-H_2O$, $CF_3COOH-H_2O$.

**. Di-O-benzyl-3,6 hexahydroxy-1,2R,3R,4R,5R,6S heptane de Formule 10 :**
F : 108°C
$(\alpha)_D$ = + 22,2° (c = 0,63, $CHCl_3$) ;
I.R. : 3950, 3760, 3700, 3560, 2990, 2690, 2300, 1410, 1250, 1060, 885, 700.
RMN : 1,31 (d, J = 7 Hz, 3H) ; 1,67 (s, 2H, OH) ; 2,59 (s, 1H, OH) ; 3,40 (s, 1H, OH) ; 3,50-4,0 (m, complexe, 7H) ; 4,48 (d., J = 14 Hz, 1H) ; 4,66 (d., J = 14 Hz, 1H) ; 4,69 (s, 2H) ; 7,30 (s, 10H).

**. Cinquième étape :**

Une solution de tétrol 10 (200 mg, 0,53 mmole) dans un mélange méthanol-eau 60/40 v/v (70 ml) contenant de l'acide acétique (0,6 ml) est agitée à température ambiante. On additionne alors goutte à goutte une solution de $NaIO_4$ (116 mg, 0,54 mmole) dans l'eau (1,2 ml). Après trois heures à température ambiante, les sels formés sont filtrés puis lavés au méthanol. Le filtrat est concentré à l'évaporateur rotatif puis le résidu est extrait de manière connue, à l'éther. On obtient ainsi un mélange des anomères α et β 11, avec un rendement quantitatif (185 mg).

. Di-O-benzyl-2,5-α (et β)-L-fucofurannose de Formule 11 :
Huile
$(\alpha)_D$ = 10,5° (c = 0,76, $CHCl_3$)
I.R. ($CH_2Cl_2$) : 3600, 3430, 2990, 2950, 2890, 1710, 1495, 1450, 1375, 1335, 1205, 1060, 1025.
RMN ($CDCl_3$) : 1,20 (d., J = 6 Hz, 3H) ; 3,5-4,7 (m. complexe dont 4,56, s, 2H) ; 5,26 (d., J = 3 hz, 1H anomère β), 5,41 (s, 1H, anomère α), 7.29 (s, 10H).

Le rapport anomère α/anomère β est voisin de 1,5 (comparaison de l'intensité des signaux à δ = 5,41 et δ = 5,26).

**. Sixième étape :**

On additionne du Pd/C à 5 % (150 mg) à une solution de dérivé benzylé 11 (742 mg, 2,15 mmoles) dans le méthanol anhydre (20 ml). La suspension est agitée sous une atmosphère d'$H_2$ pendant 6 heures, puis le palladium est filtré, lavé avec du méthanol. Le filtrat fournit, après évaporation, un mélange de α et β-L-Fucose 1a (320 mg, rendement = 91 %).

L-Fucose de Formule 1a
F : 152-153°C (recristallisé de l'éthanol)
$(\alpha)_D$ = -80° (c = 0,35, $H_2O$)
RMN $^{13}C$ : 96,30 (β,C-1) ; 92,35 (α, C-1) ; 73,16 (β, C-3) ; 71,94 (α, C-4 et β, C-2) ; 71,60 (β, C-4) ; 70,87 (β, C-5) ; 69,49 (α, C-2) ; 68,30 (α, C-3) ; 66,32 (α, C-5) ; 15,60 (α et β, C-6).
Le rapport α/β est voisin de 2/1.
RMN $^{13}C$ : (Référence : D.E. DORMAN, J.D. ROBERTS, J. Amer. Chem. Soc., 92, 1355 (1970).
β : 96,30 ; 73,00 ; 71,80 ; 71,40 ; 70,50 ; 15,60.
α 92,20 ; 71,80 ; 69,40 ; 68,20 ; 66,00 ; 15,60.

**EXEMPLE 2 : SYNTHESE DES ANALOGUES DU L-FUCOSE, AVEC R = $C_2H_5$ :**

Les analogues du L-Fucose sont préparés de manière similaire au L-Fucose.

. Première et deuxième étape :

Le D-mannose 6 est mis sous forme de diacétone mannose 7 ; un organomagnésien en solution est condensé avec le diacétone mannose 7 et fournit un mélange de 12 et 13, dans un rapport variable suivant la nature du radical R.

Dans la présente réalisation, l'organomagnésien est représenté par $C_2H_5$-MgBr et le rapport 12/13 est voisin de 4/1.

. Troisième étape :

Le mélange (3,7 g, 0,0128 mole) des deux alcools R 12b et S 13b obtenus en faisant réagir l'organomagnésien EtMgBr avec le diacétone mannose 7 dans le THF anhydre, est dissout dans la pyridine (39,5 ml). On additionne alors, sous azote, du chlorure de benzoyle (5,7 ml), puis la solution est agitée pendant 12 heures à température ambiente. Après avoir jeté le milieu réactionnel sur de l'eau glacée, une extraction à l'éther fournit un mélange des dibenzoates 14b et 15b. Ce dernier, trituré avec de l'éther de pétrole, fournit le dibenzoate cristallisé 15b (0,96g, 20 %). Les eaux-mères donnent, après évaporation, une huile homogène correspondant au dibenzoate 14b (2,60 g, 52 %).

. Di-O-benzoyl-3,6 di-O-isopropylidène-1,2,4,5 hexahydroxy 1,2R,3R,4R,5R,6R octane de Formule 15b :
F : 170°C
$(\alpha)_D$ = + 2° (c = 0,5, $CHCl_3$)
I.R. ($CH_2Cl_2$) : 2990, 1720, 1600, 1450, 1420, 1380, 1370, 1310, 1245, 1215, 1175, 1110, 1070, 1020, 890, 865.
RMN ($CDCl_3$) : 0,86 (t, J = 7 Hz, 3H) ; 1,13 (s, 3H) ; 1,27 (s, 3H) ; 1,41 (s, 3H) ; 1,60 (s, 3H) ; 4,03 (d, J = 7 Hz, 2H) ; 4,34 (q, J ; 7Hz, 1H) ; 4,47 (t, J = 7Hz, 1H) ; 4,58 (d, J = 5Hz, 1H) ; 5,07 (s large, 1H) ; 5,52 (d, J = 7Hz, 1H) ; 7,0 - 8,0 (m, 10H).

**. Di-O-benzoyl-3,6 di-O-isopropylidène-1,2,4,5 hexahydroxy-1,2R,3R,4R,5R,6S octane de Formule 14b :**
$(\alpha)_D$ = -14,5° (c = 0,5, $CHCl_3$)
I.R. ($CH_2Cl_2$) : 2980, 2940, 1715, 1600, 1450, 1360, 1310, 1270, 1250, 1170, 1110, 1060, 1020, 860.
RMN ($CDCl_3$) : 0,99 (t, J = 7Hz, 3H) ; 1,36 (s, 6H) ; 1,38 (s, 3H) ; 1,56 (s, 3H) ; 1,77 (pentuplet, J = 7 Hz, 2H) ; 4,05 (m, 2H) ; 4,3-4,6 (m, 3H) ; 5,36 (q., J = 7 Hz, 1H) ; 5,49 (d.d., $J_1$ = 3 Hz, $J_2$ = 7 Hz, 1H) ; 7,3 - 7,7 (m, 6H) ; 7,99 (d., J = 8 Hz, 1H) ; 8.06 (d., J = 8 Hz, 1H) ; 8,16 (d., J = 8 Hz, 2H).

**. Quatrième étape :**

La réaction est effectuée dans les mêmes conditions que pour la synthèse du L-Fucose. On isole ainsi le composé :

**. Di-O-benzoyl-3,6 hexahydroxy-1,2R,3R,4R,5R,6S octane de Formule 16b (rendement : 75 %) :**

F = 135°C

(α)D = - 3° (c = 0,85, $CHCl_3$)

I.R. ($CH_2Cl_2$) : 3400, 1700, 1445, 1270, 1110, 1060, 1020, 700.

RMN ($CDCl_3$) : 1,00 (t, J = 7Hz, 3H), autres signaux non caractéristiques.

**. Cinquième étape :**

Elle est réalisée selon le protocole utilisé pour le L-Fucose. On obtient les composés suivants :

**. Di-O-benzoyl-2,5 méthyl-6 α (et β)-L-Fuco-furannose de Formule 17b (Rdt quantitatif) :**

Huile

(α)D = + 36° (c = 0,8 $CHCl_3$)

I.R. : 3585, 2980, 2940, 2890, 1715, 1600, 1570, 1450, 1315, 1260, 1177, 1115, 1070, 1025.

RMN ($CDCl_3$) : 2,90 (s, large) ; 3,20 (s, large) ; 3,50 (s) ; 4,04 (t, J = 5 Hz) ; 4,18 (s large) ; 4,40 (t, J = 5 Hz) ; 5,12 (s) ; 5,43 (m) ; 5,65 (s) ; 7,3-7,6 (m, 6H) ; 7,9-8,2 (m, 4H).

Les deux isomères peuvent être caractérisés sous forme d'acétates obtenus par traitement du mélange par un excès d'anhydride acétique dans la pyridine. Une séparation chromatographique permet d'isoler les dérivés :

**. Di-O-acétyl-1,3 di-O-benzoyl-2,5 méthyl-6 β-L-Fucofurannose :**

Huile

(α)D = -40°C (c = 0,5, $CHCl_3$)

RMN ($CDCl_3$) : 1,00 (t., J = 7 Hz, 3H) ; 1,80 (pentuplet ; J = 7 Hz, 2H) ; 2,06 (s, 6H) ; 4,28 (t., J = 6 Hz, 1H) ; 5,32 (q., J = 6 Hz, 1H) ; 5,49 (d.d., $J_1$ = 6 Hz, $J_2$ = 7,5 Hz, 1H) ; 5,82 (t., J = 7,5 Hz, 1H) ; 6,52 (d., J = 6 Hz, 1H) ; 7,3-7,6 (m, 6H) ; 8,00 (d., J = 8 Hz, 2H) ; 8,10 (d., J = 8 Hz, 2H).

**. Di-O-acétyl-1,3 di O-benzyl-2,5 méthyl-6 α-L-fucofurannose :**

F : 78°C

(α)D = +29,4°C (c = 0,65, $CHCl_3$)

RMN ($CDCl_3$) : 1,00 (t., J = 7 Hz, 3H) ; 1,91 (pentuplet, J = 6 Hz, 2H) ; 2,08 (s, 3H) ; 2,17 (s, 3H) ; 4,49 (d.d., $J_1$ = 5 Hz, $J_2$ = 4 Hz, 1H) ; 5,35 (d., J = 5 Hz, 1H) ; 5,40 (s, 1H) ; 5,45 (m, 1H) ; 6,39 (s, 1H) ; 7,2 - 7,4 (m, 4H) ; 7,53 (t., J = 8 Hz, 2H) ; 7,85 (d., J = 8 Hz, 2H) ; 8,04 (d., J = 8 Hz, 2H).

**. Sixième étape :**

A une solution des dérivés benzoylés 17b (α et β) (10,9 g) dans le méthanol anhydre (185 ml), on ajoute une solution de méthylate de sodium préparée à partir de Na (2,8 g, 0,12 mole) dans le méthanol anhydre. Après 12 heures, à température ambiante, on ajoute une solution N d'acide acétique dans le méthanol (126 ml, 0,126 mole) puis on évapore le mélange réactionnel sous vide. Le résidu est repris avec du $CH_2Cl_2$ plusieurs fois puis après évaporation du $CH_2Cl_2$ le résidu cristallisé est trituré avec de l'éther-éther de pétrole 50/50 v/v, pour éliminer le benzoate de méthyle. On obtient ainsi le sucre recherché.

**- Méthyl -6 α et β-L-Fucose de Formule 1B :**

F : 130°C (déc.)

(α)D : -33° (c = 1,18, $H_2O$)

**EXEMPLE 3 : SYNTHESE DES ANALOGUES DU L-FUCOSE, AVEC R = $C_4H_9$ :**

Les étapes 1, 2 et 3 sont identiques à celles de l'Exemple 2. On isole les composés :

**. Di-O-benzoyl-3,6 di-O-isopropylidène-1,2,4,5 hexahydroxy-1,2R,3R,4R,5R,6S décane de Formule 15c (rendement : 6 %)**

F = 164°C

(α)D = -43°C (c = 1, $CHCl_3$)

I.R. ($CH_2Cl_2$) 2990, 2965, 2940, 2880, 1715, 1595, 1580, 1445, 1375, 1365, 1250, 1210, 1170, 1100, 1065, 1020, 965, 860.

RMN ($CDCl_3$) : 0,73 (t, J = 7 Hz, 3H) ; 1,18 (s, 3H) ; 1,30 (s, 3H) ; 1,43 (s, 3H) ; 1,62 (s, 3H) ; 4,04 (d., J = 6 Hz, 2H) ; 4,35 (q., J = 6 Hz, 1H) ; 4.47 (d., J = 6 Hz, 1H) ; 4,60 (d., J = 6 Hz, 1H) ; 5,10 (q., J = 6 Hz, 1H) ; 5,56 (d., J = 6 Hz, 1H) ; 7,3-7,7 (m, 6H) ; 8-8,3 (m, 4H).

**. Di-O-benzoyl-3,6 di-O-isopropylidene-1,2,4,5 hexahydroxy-1,2R,3R,4R,5R, 6S décane de Formule 14c (91 %) :**

**Hulle**

(α)D : -5° (c = 1,32, $CHCl_3$)

I.R. ($CH_2Cl_2$) : 2990, 2965, 2940, 2880, 1715, 1595, 1580, 1445, 1375, 1365, 1250, 1210, 1170, 1100, 1065, 1020, 965.

RMN ($CDCl_3$) : 0,90 (t, J = 7Hz, 3H) ; 1,38 (s, 9H) ; 1,58 (s, 3H) ; 1,74 (m, 2H) ; 4,06 (m, 2H) ; 4,32 (t., J = 6 Hz, 2H) ; 4,35 - 4,55 (m, complexe) ; 5,50 (m, 2H).

**. Quatrième étape :**

Le protocole est identique à celui de l'exemple 2 ; on isole le composé suivant :

**. Di-O-benzoyl-3,6 hexahydroxy-1, 2R, 3R, 4R, 5R, 6S décane de Formule 16c (rendement : 56 %)**

F : 138°C.

$(\alpha)_D$ : -27°C (c = 1,$CHCl_3$)

I.R. ($CHCl_3$) : 3450, 3020, 2970, 2940, 1695, 1600, 1450, 1280, 1180, 1115, 1070, 1025, 705.

RMN ($CDCl_3$) : Signaux non caractéristiques sauf 0,87 (t, 3H).

**. Cinquième étape :**

Elle est réalisée selon le protocole utilisé pour le L-fucose. On obtient ainsi le composé suivant :

**. Di-O-benzoyl-2,5-propyl-6 α et β-L-Fucofurannose 17c (Rdt quantitatif) :**

Huile

$(\alpha)_D$ : + 3,1° (c = 1, $CHCl_3$)

I.R. ($CH_2Cl_2$) : 3585, 2960, 2940, 2880, 1705, 1595, 1577, 1445, 1310, 1260, 1170, 1110, 1065, 1020.

RMN ($CDCl_3$) : 0,83 (t. 3H) ; 1,33 (s large, 4H) ; 1,82 (s large, 2H) ; 2,00 (s, 1H) ; 3,6-3,85 (m) ; 4,10 (t, J = 6 Hz) ; 4,28 (d., J = 4Hz) ; 4,54 (t., J = 4 Hz) ; 4,64 (t., J = 6Hz) ; 5,0-5,75 (massif complexe dont 5,26 (s) et 5,64 (s) ; 7,2-7,6 (m, 6H) ; 7,8-8,2 (m, 4H).

Les intensités des signaux ne sont pas données (mélange des anomères α et β).

**. sixième étape :**

. Propyl-6 α et β-L-Fucose de formule 1c

F : 140°C (dec.)

$(\alpha)_D$ : -30°C (c = 1, $H_2O$)

**EXEMPLE 4 : SYNTHESE DES GLYCALS, AVEC R = $C_2H_5$ :**

**. Première étape :**

Le méthyl-6 Fucose (5g, 0,028 mole) est mis en suspension dans la pyridine (50 ml) et l'anhydride acétique (25 ml). Le mélange réactionnel est agité pendant 48 heures à température ambiante. La solution obtenue est jetée sur de l'eau glacée puis extraite de la manière habituelle avec $CH_2Cl_2$. On obtient 4,95 g (Rdt : 51 %) d'un mélange de tétraacétates 18b α et 18b β).

Huile

$(\alpha)_D$ = -32,4° (c = 1,11, $CHCl_3$)

I.R. ($CH_2Cl_2$) : 2990, 1750, 1370, 1220, 1090, 1060, 900.

RMN ($CDCl_3$) : 0,92 (t, J = 7Hz, 1H) ; 1,5 - 1,8 (m, 2H) ; 1,99 (s, 3H) ; 2,04 (s, 3H) ; 2,12 (s, 3H) ; 2,18 (s, 3H) ; 3,65 (t, J = 6,5Hz, 1H) ; 5,06 (d.d., $J_1$ = 3,5Hz, $J_2$ = 10Hz, 1H) ; 5,2 - 5,4 (m, 2H) ; 5,66 (d, J = 8Hz, 1H).

**. Deuxième étape :**

Une solution de diacétates 18b α et β (5g) dans l'acide acétique (1,25 ml) et l'anhydride acétique (1,25 ml) est maintenue sous agitation à 0°C. On ajoute alors goutte à goutte une solution de HBr à 30 % dans l'acide acétique (9 ml) puis on agite jusqu'à ce que la température soit revenue à 20°C.

Cette solution est alors additionnée lentement à une solution refroidie à -10°C d'acétate de sodium (12,5 g) dans l'acide acétique-eau 50/50 v/v (30 ml) à laquelle on a préalablement ajouté (à cette température) une solution de $CuSO_4$, $5H_2O$ (0,9 g) dans 2,75 ml d'eau et du zinc pulvérisé (9g). Après la fin de l'addition, l'agitation est maintenue pendant deux heures à -10°C.

On filtre alors sur célite, on lave deux fois avec une solution de $CH_3COOH$-$H_2O$, puis on extrait avec $CH_2Cl_2$. Après lavage avec une solution saturée de bicarbonate, puis de l'eau et enfin séchage sur $Na_2SO_4$, on obtient une huile qui est purifiée par filtration rapide sur gel de silice (éluant éther-éther de pétrole 70/30).

On obtient ainsi le glycal de Formule 2b (2,43 g, Rdt : 73 %)

F : 47-48°C

$(\alpha)_D$ = + 3,7° (c = 1,06, $CHCl_3$)

I.R. ($CH_2Cl_2$) : 3000, 2900, 1745, 1655, 1375, 1240, 1155, 1100, 1080, 1035, 910.

RMN ($CDCl_3$) : 0,97 (t., J = 7 Hz, 3H) ; 2,01 (s, 3H) ; 3,93 (t., J = 7 Hz, 1H) ; 4,65 (d.t., $J_1$ = 6Hz, $J_2$ = 2 Hz, 1H) ; 5,37 (d., J = 4 Hz, 1H) ; 5,56 (s large, 1H) ; 6,46 (d.d., $J_1$ = 6Hz, $J_2$ = 2 Hz, 1H).

**EXEMPLE 5 ; SYNTHESE DES GLYCALS EN SERIE BUTYLE, AVEC R = $C_4H_9$ :**

**. Première étape :**

La réaction d'acétylation est réalisée dans les mêmes conditions que dans l'exemple 4. On isole ainsi un mélange des tétraacétates 18c α et 18c β, sous forme d'huile.

De ce mélange peut être recristallisé en partie dans l'éther le tétraacétate β de Formule 18c.

F : 111°C

$(\alpha)_D$ = -31° (c = 1, $CHCl_3$)

I.R. : 3070, 2980, 2890, 1750, 1370, 1220, 1095, 1065, 1050.

RMN ($CDCl_3$) : 0,86 (t., J = 7 Hz, 3H) ; 1,29 (m, 4H) ; 1,99 (s, 3H) ; 2,04 (s, 3H) ; 2,11 (s, 3H) ; 2,17 (s, 3H) ; 3,74

(d.d., $J_1$ = 8Hz, $J_2$ = 3,5 Hz, 1H) ; 5,05 (d.d., $J_1$ = 3,5 Hz, $J_2$ = 10 Hz, 1H) ; 5,29 (d., J = 9Hz, 1H) ; 5,34 (d., J = 8 Hz, 1H) ; 5,47 (d., J = 9 Hz, 1H).

**. Deuxième étape :**

La réaction est effectuée comme dans l'exemple 4. On obtient le glycal de Formule 2c (Rdt : 60 %).
Huile
$(\alpha)_D$ = -24° (c = 1,68, $CHCl_3$)
I.R. ($CH_2Cl_2$) : 2970, 2950, 2845, 1740, 1375, 1230, 1150, 1120, 1050.
RMN ($CDCl_3$) : 0,98 (t., J = 7 Hz, 3H) ; 1,34 (m) ; 2,01 (s, 3H) ; 3,99 (d.d., $J_1$ = 8Hz, $J_2$ = 4 Hz, 1H) ; 4,63 (d.t., $J_1$ = 6 Hz, $J_2$ = 2 Hz, 1H) ; 5,33 (d., J = 4 Hz, 1H) ; 5,56 (s large, 1H) ; 6,47 (d.d., $J_1$ = 8 Hz, $J_2$ = 2 Hz, 1H).

**EXEMPLE 6 ; GLYCOSIDATION DE LA BETA-RHODOMYCINONE AVEC UN RADICAL DE FORMULE 19′ DANS LEQUEL R EST UN GROUP $CH_3$ :**

A une solution de β-Rhodomycinone (300 mg, 0,78 mmole) dans le chlorure de méthylène anhydre (150 ml) on additionne successivement HgO (1,014 g), $HgBr_2$ (297 mg) puis des tamis moléculaires 3 Å (3g).

Cette suspension est agitée sous azote à température ambiante pendant l'addition d'une solution de chloro-1 di-O-acétyl-3,4-tridésoxy-1,2,6-L-Galactose (19 a) (488 mg) dans $CH_2Cl_2$ (15 ml).

Après 12 heures à température ambiante le mélange réactionnel est filtré (la fraction insoluble est lavée avec $CH_2Cl_2$) puis extrait.

La phase organique est lavée successivement avec une solution de bicarbonate puis de l'eau. Après séchage, on obtient une huile qui est chromatographiée sur gel de silice (éluant : $CH_2Cl_2$-MeOH-AcOH : 99-1-0,2 v/v).

On isole ainsi successivement le bisglycoside en positions 7 et 10 (10-15 %) puis le glycoside en position 7 (40-50 %).

**. Di-O-(di-O-acétyl-3′,4′ didésoxy-2′,6′-α-L-thréo-hexopyrannosyl) - 7,10 β-Rhodomycinone de Formule 22a.**
F : 157-158°C
$(\alpha)_D$ = + 399° ($CHCl_3$)
I.R. ($CHCl_3$) : 3680, 3340, 2980, 2940, 1735, 1600, 1570, 1435, 1395, 1360, 1275, 1245, 1230, 1195, 1160, 1110, 1075, 1010, 955.
RMN ($CDCl_3$) : 1,11 (t, J = 7 Hz, 3H) ; 1,92 (s, 6H) ; 2,15 (s, 3H) ; 2,18 (s, 3H) ; 3,32 (s, 1H, OH) ; 4,16 (s., J = 6,5 Hz, 1H) ; 4,29 (q., J = 6,5 Hz, 1H) ; 5,01 (s, 1H) ; 5,0-5,3 (m complexe, 4H) ; 5,53 (d., J = 2,5 Hz, 2H) ; 7,32 (d., J = 8 Hz, 1H) ; 7,72 (t., J = 8 Hz, 1H) ; 7,89 (d., J = 8 Hz, 1H) ; 12,12 (s, 1H, OH) ; 12,87 (s, 1H, OH) ; 13,73 (s, 1H, OH).

**. O-(di-O-acétyl-3′,4′ didésoxy-2′,6′-α-L-thréo-hexopyrannosyl)-7 β-Rhodomycinone de Formule 21a :**
F : 136 - 140°C
$(\alpha)_D$ = + 174° ($CHCl_3$)
I.R. ($CH_2Cl_2$) : 3700, 3600, 2990, 2940, 1735, 1600, 1575, 1415, 1360, 1230, 1200, 1160, 1075, 1035, 1015, 980, 960.
RMN ($CDCl_3$) : 1,13 (t., J = 7 Hz, 3H) ; 1,23 (d., J = 6 Hz, 1H) ; 1,92 (s, 3H) ; 2,18 (s, 3H) ; 3,67 (s, 1H) ; 4,35 (q., J = 6 Hz, 1H) 4,89 (s, 1H) ; 5,10 (s large, 1H) ; 5,21 (s, 1H) ; 5,54 (s, 1H) ; 7,30 (d., J - 8 Hz, 1H) ; 7,69 (t, J = 8 Hz, 1H) ; 7,86 (d., J = 8hz, 1H) ; 12,09 (s, 1H) ; 12,89 (s, 1H) ; 13,55 (s, 1H).

**EXEMPLE 7 : GLYCOSIDATION DE LA β-RHODOMYCINONE, AVEC UN RADICAL DE FORMULE 19′, DANS LEQUEL R EST UN GROUPE ETHYLE :**

La réaction est effectuée comme dans l'exemple 6, en utilisant le chloro-1 di-O-acétyl-3,4-méthyl-6 tridésoxy-1,2,6-L-Galactose 19b.

On obtient ainsi :

**. le Di-O-(di-O-acétyl-3′,4′tridésoxy-2′,6′,7′-α-L-thréo heptopyrannosyl)-7,10 β-Rhodomycinone de Formule 22b.**
F : 196 - 199° (dec)
$(\alpha)_D$ = + 464° ($CHCl_3$, c = 1,66)
I.R. ($CHCl_2$) : 3700, 3540, 2990, 2940, 1735, 1600, 1575, 1415, 1365, 1270, 1240, 1200, 1160, 1115, 1060, 1015, 985, 970.
RMN ($CDCl_3$) : 1,93 (s, 6H) ; 2,14 (s, 3H) ; 2,18 (s, 3H) ; 3,31 (s, 1H, OH) ; 3,89 (t., J = 7 Hz, 1H) ; 4,01 (t., J= 7Hz, 1H) ; 4,99 (s, 1H) ; 5-5,25 (m complexe, 4H) ; 5,29 (s, 1H) ; 5,50 (d., J = 2,5 Hz, 1H) ; 5,58 (d., J = 2,5 Hz, 1H) ; 7,32 (d., J= 8Hz, 1H) ; 7,72 (t., J = 8Hz, 1H) ; 7,89 (d., J = 8 Hz, 1H) ; 12,09 (s, 1H, OH) ; 12,88 (s, 1H, OH) ; 13,69 (s, 1H, OH).

**. le O-(di-O-acétyl-3′-4′ tridésoxy-2′,6′,7′-α-L-thréo-heptopyrannosyl)-7 β-Rhodomycinone de Formule 21b :**
F : 133-135°C (dec.)
$(\alpha)_D$ = + 208°C (c = 1,96, $CHCl_3$)
I.R. ($CH_2Cl_2$) : 3700, 3540, 3000, 2950, 1715, 1610, 1580, 1420, 1360, 1250, 1220, 1170, 1025.
RMN ($CDCl_3$) : 1,03 (t., J = 7 Hz, 3H) ; 1,13 (t., J = 7 Hz, 3H) ; 1,93 (s, 3H) ; 2,18 (s, 3H) ; 3,15 (s, large, 1H, OH) ; 3,76 (s, 1H, OH) ; 4,10 (t., J = 7 Hz, 1H) ; 4,85 (s, 1H) ; 5,07 (d., J = 2 Hz, 1H) ; 5,32 (s, 1H) ; 7,63 (t., J = 8 Hz, 1H) ; 5,58 (d., J = 2Hz, 1H) ; 7,20 (d., J = 8 Hz, 1H) ; 7,73 (d., J = 8 Hz, 1H) ; 11,94 (s, 1H, OH) ;

12,75 (s, 1H, OH) ; 13,40 (s, 1H, OH).

EXEMPLE 8 : GLYCOSIDATION DE LA β-RHODOMYCINONE, AVEC UN RADICAL DE FORMULE 19′, DANS LEQUEL R EST UN GROUPE BUTYLE :

La réaction est effectuée comme dans l'exemple 6, en utilisant le chloro-1 di-O-acétyl-3,4 propyl-6 tridésoxy-1,2,6,-L-Galactose 19c.

On obtient ainsi :

**. le di-O-(di-O-acétyl-3′,4′ hexadésoxy-2′,6′,7′,8′,9′, 10′-α-L-thréo décapyrannosyl)-7,10 β-Rhodomycinone de Formule 22 c :**

F : 190 - 192°C (changement d'état : 163-167°C)

$(\alpha)_D = + 320°$

I.R. ($CH_2Cl_2$) : 3710, 3580, 2980, 2960, 2890, 1740, 1605, 1585, 1465, 1410, 1375, 1245, 1205, 1170, 1130, 1070, 1025, 1000, 980.

RMN ($CDCl_3$) : 1,94 (s, 6H) ; 2,17 (s, 6H) ; 3,31 (s, 1H) ; 3,9-4,2 (M, 2H) ; 5,01 (s, 1H) ; 5,0-5,45 (m, 5H) ; 5,50 (s large, 1H) ; 5,60 (s large, 1H) ; 7,32 (d, J = 8 Hz, 1H) ; 7,72 (t., J = 8 Hz, 1H) ; 7,92 (d., J = 8 Hz, 1H) ; 12,13 (s, 1H) ; 12,91 (s, 1H) ; 13,70 (s, 1H).

**. le O-(di-O-acétyl-3′,4′hexadésoxy-2′,6′,7′,8′,9′,10′-α-L-thréo décapyrannosyl)-7 β-Rhodomycinone de Formule 21c :**

F : 110-112°C

$(\alpha)_D$ : + 179° ($CHCl_3$)

I.R. ($CH_2Cl_2$) : 3580, 3530, 2960, 2920, 2860, 1735, 1600, 1575, 1455, 1410, 1360, 1240, 1190, 1160, 1130, 1115, 1065, 1020, 990, 965.

RMN ($CDCl_3$) : 0,94 (t., J = 6 Hz, 3H) ; 1,13 (t., J = 7 Hz, 3H) ; 1,94 (s, 3H) ; 2,17 (s, 3H) ; 3,73 (s, 1H, OH) ; 4,14 (s large, 1H) ; 4,90 (s, 1H) ; 5,13 (s large, 1H) ; 5,29 (s, 1H) ; 5,59 (d., J = 2 Hz, 1H) ; 7,32 (d., J = 8 Hz, 1H) ; 7,79 (t., J = 8 Hz, 1H) ; 7,84 (d., J = 8 Hz, 1H) ; 12,09 (s, 1H, OH) ; 12,84 (s, 1H, OH) ; 13,55 (s, 1H, OH).

**EXEMPLE 9 : GLYCOSIDATION DE LA DAUNOMYCINONE :**

Elle est effectuée selon le même mode opératoire que celui utilisé pour la β-Rhodomycinone (exemple 5 à 8).

**- série éthyle :**

On obtient l'acétoxy-3′ O-acétyl-4′ désamino-3′ méthyl-6′daunorubicine de Formule 20b.

F : 126-131°C

$(\alpha)_D = 216°$ (c = 0,27, $CHCl_3$)

I.R. ($CH_2Cl_2$) : 3500, 1730, 1710, 1620, 1580, 1425, 1380.

RMN ($CDCl_3$) : 0,99 (t., 3H) ; 1,95 (s, 3H) ; 2,16 (s, 3H) ; 2,43 (s, 3H) ; 2,92 (d., J = 19 Hz, 1H) ; 3,25 (d, J = 19 Hz, 1H) ; 3,98 (t., 1H) ; 4,10 (s, 3H) ; 4,40 (s, 1H) ; 5,05 (d.m., 1H) ; 5,30 (s large, 2H) ; 5,63 (d., J = 3 Hz, 1H) ; 7.40 (d., 1H) ; 7,79 (t., 1H) ; 8,05 (d., 1H) ; 12,53 (s., 1H) ; 13,27 (s, 1H).

**- série Butyl :**

On obtient l'acétoxy-3′ O-acétyl-4′ désamino-3′ propyl-6′ daunorubicine de Formule 20c.

F : non mesurable (pas de point de fusion défini).

$(\alpha)_D = 226°$ (c = 0,2, $CHCl_3$)

I.R. ($CH_2Cl_2$) : 3500, 2960, 2940, 1720, 1705, 1605, 1425, 1375.

RMN ($CDCl_3$) : 0,91 (t., 3H) ; 1,94 (s. 3H) ; 2,17 (s, 3H) ; 2,43 (s, 3H) ; 2,84, (d., J = 19 Hz) ; 3,19 (d., J = 19 Hz) ; 4,09 ($OCH_3$, 3H) ; 4,39 (s large) ; 5,06 (d.m., 1H) ; 5,29 (s large, 2H) ; 5,64 (d. large, J = 3,2 Hz, 1H) ; 7,40 (d., 1H) ; 7,78 (t., 1H) ; 8,01 (d., 1H) ; 11,60 (s, 1H) ; 12,36 (s, 1H).

**COMPTE RENDU PHARMACOLOGIQUE CONCERNANT DES GLYCOSIDES SELON L'INVENTION.**

**I - TEST DE PROLIFERATION :**

A - Protocole opératoire (réduction MTT) :

Des cellules tumorales L1210, A549 et HT29, à une concentration de $5.10^3$/ml dans un milieu RPMI, sont incubées dans des plaques de microtitration contenant 96 puits, pendant 72 heures (37°C, 5 % $CO_2$, 95 % d'humidité relative) avec différentes concentrations de chacun des glycosides (anthracyclines) conformes à l'invention.

Les témoins consistent en cellules tumorales exposées à un milieu de culture. Quatre puits sont préparés pour chaque concentration en glycoside et pour le témoin. Après 65 heures, 50 µl de MTT (2,5 mg/ml dans du PBS) sont ajoutés.

Le MTT sera réduit, en présence de cellules vivantes en un colorant formazan rouge insoluble. Après 7 à 24 heures d'incubation supplémentaire, le surnageant est enlevé. Le colorant formazan est solubilisé par l'addition de 100 µl de DMSO dans chaque puits, suivie d'une agitation douce.

L'extinction est mesurée pour chaque puits, à 492 nm (photomètre Multiscan 340 CC Fa. Flow).

B - Résultats :

Les résultats sont exprimés comme le rapport de l'extinction après incubation avec les glycosides sur l'extinction obtenue avec les témoins. Le coefficient de variation est inférieur à 15 %.

Les résultats sont représentés dans le tableau I ci-après.

Les produits testés sont les suivants :

TABLEAU I

| | $CI_{50}$ (µg/ml) TEST AU MTT | | |
|---|---|---|---|
| | L1210 | HT 29 | A 549 |
| 21 b | 0,51 | > 1 | 0,46 |
| 22 b | > 1 | > 1 | > 1 |
| 21 c | > 1 | > 1 | > 1 |
| 22 c | > 1 | > 1 | > 1 |
| 20 b | >1/>10 | 0,35/>10 | >1/>10 |
| 20 c | > 1 | > 1 | > 1 |
| 21 a | 0,03 | 0,038 | 0,086 |

**II - EFFET SUR LES CELLULES LEUCEMIQUES L1210 :**

A - Protocole opératoire :

L'essai est réalisé selon la procédure de Hamburger et Salmon, avec les quelques modifications précisées ci-après.

Le milieu utilisé est remplacé par un milieu de McCoy 5A. Le nombre de cellules mises dans les boîtes est réduit à $5.10^2$ cellules/boîte du fait de la croissance rapide des cellules leucémiques L 1210.

Les cellules sont incubées pendant 1 heure à 37°C en présence de différentes concentrations de la substance testée. Puis les cellules sont lavées deux fois à l'aide de McCoy 5A et mises sur plaque d'agarose selon la méthode de Hamburger et Salmon.

Par ailleurs, le test est réalisé parallèlement avec incubation continue avec différentes concentrations de la substance étudiée en introduisant ladite substance dans la couche d'agarose avant la mise en culture des cellules.

Les boîtes sont mises à l'étuve à 37°C sous une atmophère de 5 % $CO_2$, 20 % $O_2$ et une humidité relative de 95 %, pendant 5 à 7 jours. Après cette période, les colonies de diamètre supérieur à 60 µm sont comptées à l'aide d'un microscope inversé.

B - Résultats :

Les résultats résumés dans le Tableau II, ci-après sont exprimés en pourcentage de colonies formées à partir des cellules L 1210 traitées par rapport aux témoins non traités. Le coefficient de variation, lors de la répétition des expériences, est inférieur à 15 %.

TABLEAU II

| | ESSAI SUR CELLULES LEUCEMIQUES | | | | | |
|---|---|---|---|---|---|---|
| | expérience en continu | | | expérience d'une heure | | |
| | L1210 | HT 29 | A 549 | L1210 | HT 29 | A 549 |
| 21 b | | | | 0,8 | | |
| 21 a | 0,057 | | | 0,38 | | |
| 22 a | 1,2 | | | > 1 | | |

**III - ETUDE DE L'EFFET DU COMPOSE 21a, *IN VIVO* :**

A - Protocole opératoire :

Préparation des cellules L1210 :

Un liquide d'ascite est prélevé dans les conditions aseptiques chez des souris DBA2 (femelle, 18 - 20 g) le septième jours après l'implantation.

L'ascite est lavée trois fois avec du PBS, comptée et finalement diluée dans du PBS, de manière à obtenir

$10^6$ cellules/0,2 ml.

- Transfert des cellules pour l'entretien de la lignée cellulaire :
  $10^6$ cellules dans 0,2 ml de P BS sont injectées dans le peritoine de souris DBA2 pour la propagation de la lignée cellulaire.
  Le transfert est effectué une fois par semaine.

- Transfert des lignées cellulaires pour le test :
  $10^6$ cellules dans 0,2 ml de PBS sont injectés intrapériotonéalement à des souris BDF1 (femelle 18 - 20 g), 6 animaux/group sont utilisés pour chaque concentration de substance et pour le contrôle.

B - Evaluation de l'effet :

a) les animaux sont pesés le premier jour et le cinquième jour après l'administration du médicament. Une perte de poids supérieure à 20 % le cinquième jour est utilisée comme indicateur d'effet toxique du produit.

b) à la fin de l'expérience, (mort de tous les animaux ou soixantième jour de l'essai), le temps moyen de survie des animaux, dans tous les groupes ayant plus de 65 % de survivants au cinquième jour, est évalué selon des procédures standard.

Les animaux survivants au soixantième jour sont considérés comme des survivants de longue durée (LTS : *Long Time Survivors*) et sont répertoriés séparément.

A partir du temps de survie moyen des groupes traités ($MST_T$) et des groupes contrôle ($MST_C$), l'effet antitumoral (T/C) est évalué en fonction de la formule suivante :

$$T/C\ \% = \frac{MST_T}{MST_C} \times 100$$

Les valeurs T/C de plus de 125 % sont considérées comme des indicateurs d'un effet antitumoral significatif.

L'effet antitumoral est précisé dans le Tableau III, ci-après :

TABLEAU III

| nom | programme d'administration | dose optimale (mg/kg/Inj.) | L1210 | | Morts toxiques |
|---|---|---|---|---|---|
| | | | T/C % | LTS* | |
| 21a | 3 x 1.p./ 1.p. Q3D | 10.00 | 134 | - | - |
| | | 13.30 | 152 | - | - |
| | | 17.70 | 146 | - | 1/6 |

* LTS : Survivant à long terme.

**IV - ETUDE DE LA TOXICITE AIGUE DU COMPOSE 21a :**

La substance à tester est injectée à des souris NMRI selon le protocole tel que précisé dans le Tableau IV, ci-après.

Après deux semaines, le nombre total d'animaux décédés dans chaque groupe est calculé et les $DL_{50}$ sont évaluées en utilisant la méthode de Litchfield Wilcoxon.

Les résultats sont présentés dans le Tableau IV, ci-après :

TABLEAU IV

| Substance | programme d'administration | $DL_{50}$ |
|---|---|---|
| 21a | 3 x i.p. | > 21.3 |

Les médicaments contenant les anthracyclines conformes à la présente invention sont généralement administrés à des doses comprises entre 0,001 et 25 mg/kg/jour.

Ils sont particulièrement adaptés au traitement des leucémies aiguës et chroniques, à la maladie de Hodgkin, aux lymphomes non-hodgkiniens.

Ainsi que cela ressort de ce qui précède, l'invention ne se limite nullement à ceux de ses modes de réalisation, de mise en oeuvre et d'application qui viennent d'être décrits de façon plus explicite ; elle en embrasse au contraire toutes les variantes qui peuvent venir à l'esprit du technicien en la matière, sans s'écar-ter du cadre, ni de la portée de la présente invention.

**Revendications**

1°) Analogues du L-Fucose, correspondant à la formule générale 1′

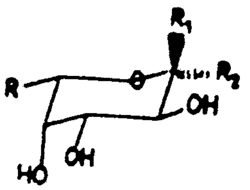

(1′)

dans laquelle :

**R** représente un groupe alkyle éventuellement substitué, linéaire ou ramifié ou un groupe aryle éventuellement substitué, à l'exception d'un groupe méthyle, d'un groupe $CH_2OH$ ou d'un groupe $CH_2OR_3$, $R_3$ ayant la même signification que R ;

**$R_1$ et $R_2$** sont toujours différents et représentent un atome d'hydrogène ou un group hydroxyle, étant donné que lorsque **$R_1$** est un atome d'hydrogène et $R_2$ est un groupe OH, on obtient un stéréoisomère β et lorsque $R_1$ est un groupe hydroxyle et $R_2$ est un atome d'hydrogène, on obtient un stéréoisomère α.

2°) Procédé de préparation du L-Fucose de Formule 1, et/ou de ses analogues de Formule 1′, selon la revendication 1, à partir de D-mannose, caractérisé :

a. en ce qu'on transforme le D-mannose de Formule 6 en diacétone mannose de Formule 7,

b. en ce qu'on condense un dérivé organométallique de formule R-Y, dans laquelle :

**R** est un groupe alkyle linéaire ou ramifié insaturé ou non, éventuellement substitué ou un groupe aryle substitué ou non, y compris un groupe méthyle et

**Y** du magnésium ou du lithium

avec le diacétone mannose de Formule 7, ladite condensation fournissant :

- le diol de Formule 8, dans le cas où R représente un groupe méthyle, et

c. en ce qu'on protège les fonctions alcool dudit diol de Formule 8 par un traitement par un composé de formule R′-X, dans laquelle :

**R′** est un groupe benzyle éventuellement substitué et

**X** un halogène

pour obtenir le composé de Formule 9,

d. en ce qu'on hydrolyse, au cours d'une quatrième étape, le composé de Formule 9 pour donner le produit de Formule 10,

e. en ce qu'on scinde le produit de Formule 10, au niveau du glycol en bout de chaîne, au cours d'une cinquième étape pour otenir un mélange de deux anomères α et β de Formule 11,

f. en ce que l'on transforme le mélange obtenu au cours de l'étape e. en produit recherché (1), au cours d'une sixième étape, par hydrogènolyse,

selon le schéma I suivant :

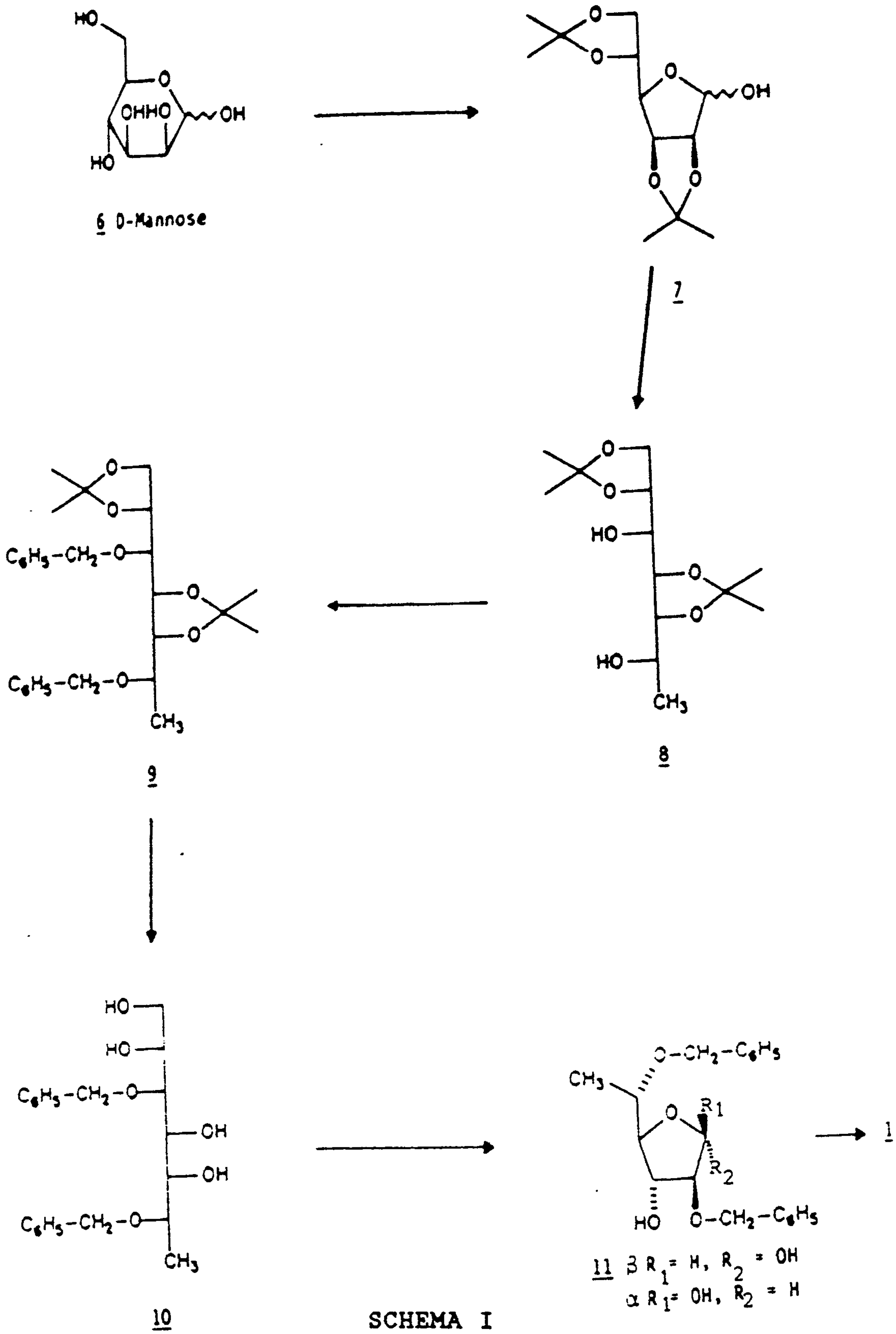

6 D-Mannose

7

8

9

10

11 β $R_1$ = H, $R_2$ = OH
α $R_1$ = OH, $R_2$ = H

1

SCHEMA I

- un mélange de deux diols 12 et 13, dans le cas où R représente un groupe alkyle éventuellement

substitué, linéaire ou ramifié ou un groupe aryle éventuellement substitué, à l'exception d'un groupe méthyle, d'un groupe $CH_2OH$ ou d'un groupe $CH_2OR_3$, $R_3$ ayant la même signification que R, et
c. en ce qu'on protège les fonctions alcool dudit mélange de diols 12 et 13 par un traitement par un composé de formule R'-X, dans laquelle :
**R'** est un groupe benzyle éventuellement substitué et
**X** un halogène
pour obtenir les composés de Formules 14 et 15,
d. en ce qu'on hydrolyse, au cours d'une quatrième étape, le composé 14 pour donner le produit de Formule 16,
e. en ce qu'on scinde le produit de Formule 16, au niveau du glycol en bout de chaîne, au cours d'une cinquième étape pour obtenir un mélange de deux anomères α et β de Formule 17,
f. en ce que l'on transforme le mélange obtenu au cours de l'étape e. en produit recherché (1'), au cours d'une sixième étape, par hydrolyse,
selon le schéma II suivant :

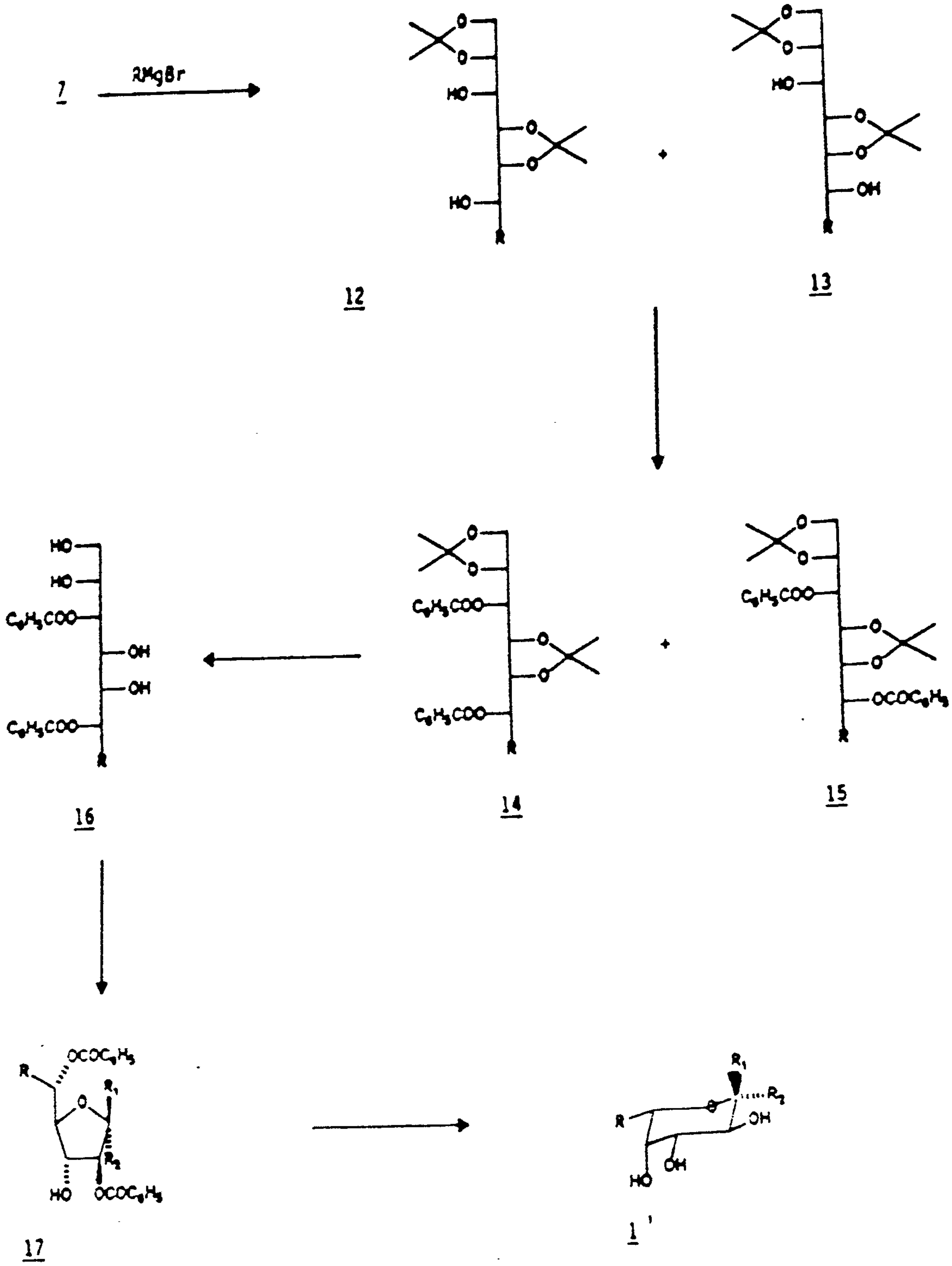


7 12 13 14 15 16 17 1'

SCHEMA II

3°) Glycals à partir du L-Fucose et/ou de ses analogues selon la revendication 1, de Formule 2:

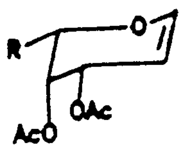

(2)

dans laquelle :
**R** représente un groupe alkyle éventuellement substitué, linéaire ou ramifié ou un groupe aryle éventuellement substitué, à l'exception d'un groupe $CH_2OH$ ou d'un groupe $CH_2OR_3$, $R_3$ ayant la même signification que R.

4°) Procédé de préparation de nouvelles anthracyclines à partir d'un glycal selon la revendication 3, caractérisé en ce que :

a. ledit glycal de Formule 2 est transformé en dérivé halogéné par addition d'un composé de Formule HX anhydre, dans laquelle X est un halogène, pour obtenir un composé de Formule 19, selon le schéma IV :

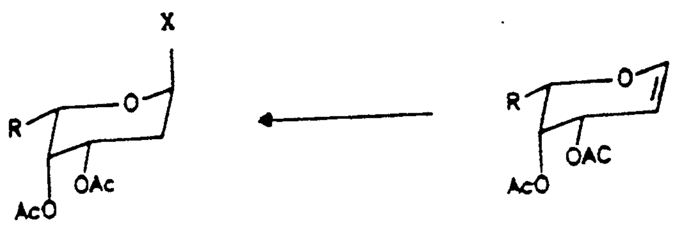

19 SCHEMA IV 2

b. puis ledit composé de Formule 19 est couplé à une anthracyclinone appropriée, par une réaction de type Koenigs-Knorr.

5°) Procédé selon la revendication 4, caractérisé en ce que ledit composé 19 est couplé avec la daunomy cinone et fournit une anthracycline de Formule 20' :

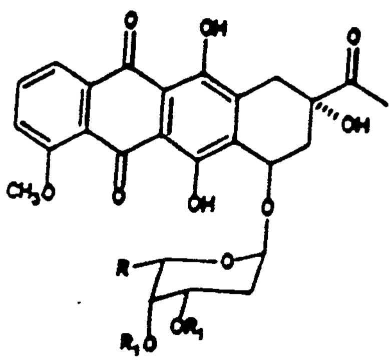

(20')

dans laquelle :
**R** est tel que défini ci-dessus ;
**$R_1$** représente un groupe acétyle ou un atome d'hydrogène.

6°) Procédé selon la revendication 4, caractérisé en ce que ledit composé 19 est couplé avec la β rhodomycinone et fournit une anthracycline de Formule 21'.

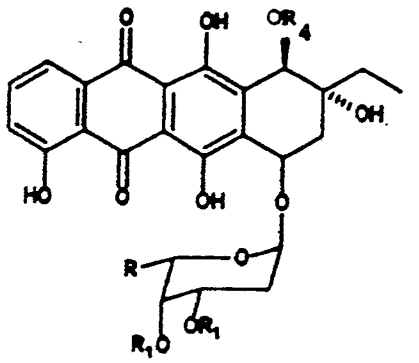

(21')

dans laquelle,
**R** est tel que défini ci-dessus ;
**$R_1$** représente un groupe acétyle ou un atome d'hydrogène ;
**$R_4$** représente un atome d'hydrogène ou un radical de Formule 19',

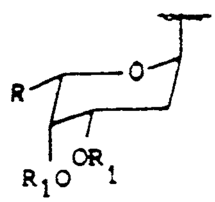

(19')

dans lequel R et $R_1$ ont les mêmes significations que précédemment.

7°) Procédé selon l'une quelconque des revendications 4 à 6, caractérisé en ce que les glycosides obtenus sont purifiés par chromatographie.

8°) Anthracycline, caractérisée en ce qu'elle est obtenue par le procédé selon les revendications 4 à 7 et en ce qu'elle est de Formule 20', ci-après :

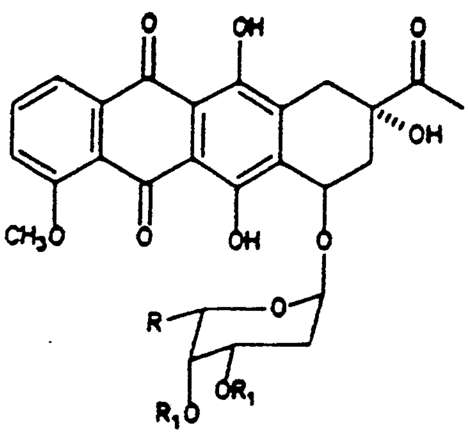

(20')

dans laquelle :
**R** est tel que défini ci-dessus ;
**$R_1$** représente un groupe acétyle ou un atome d'hydrogène.

9°) Anthracycline, caractérisée en ce qu'elle est obtenue par le procédé selon les revendications 4 à 7 et en ce qu'elle est de Formule 21', ci-après :

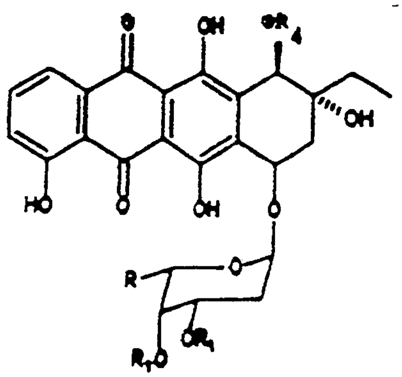

(21')

dans laquelle,
**R** est tel que défini ci-dessus ;
**$R_1$** représente un groupe acétyle ou un atome d'hydrogène ;
**$R_4$** représente un atome d'hydrogène ou un radical de For mule 19',

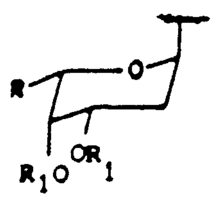

(19')

dans lequel R et $R_1$ ont les mêmes significations que précédemment.

10°) Anthracycline selon la revendication 9 caractérisée en ce qu'elle est constituée par la Di-O-(di-O-acétyl-3',4' didésoxy-2',6'-α-L-thréo-hexopyrannosyl) -7,10 β-Rhodomycinone de Formule 22a, ci-après.

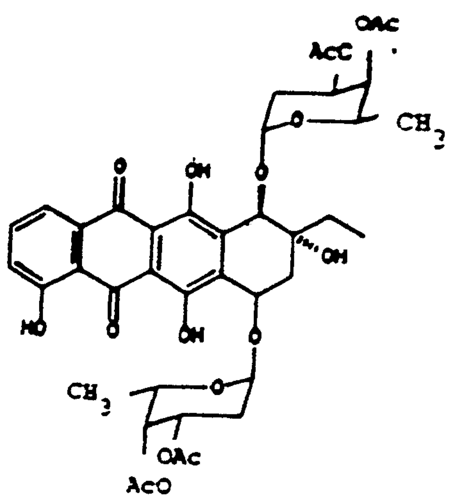

(22a)

11°) Anthracycline selon la revendication 9, caractérisée en ce qu'elle est constituée par la O-(di-O-acétyl-3',4' didésoxy-2'6'-α-L-thréo-hexopyrannosyl)-7 β-Rhodomycinone de Formule 21a, ci-après :

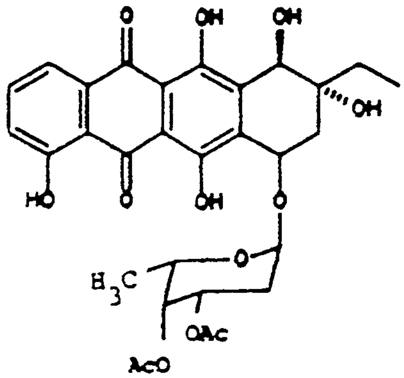

(21a)

12°) Anthracycline selon la revendication 9, caractérisée en ce qu'elle est constituée par la Di-O-(di-O-acétyl-3′,4′ tridésoxy-2′,6′ ,7′-α-L-thréo heptopyranno syl)-7,10 β-Rhodomycinone de Formule 22b, ci-après :

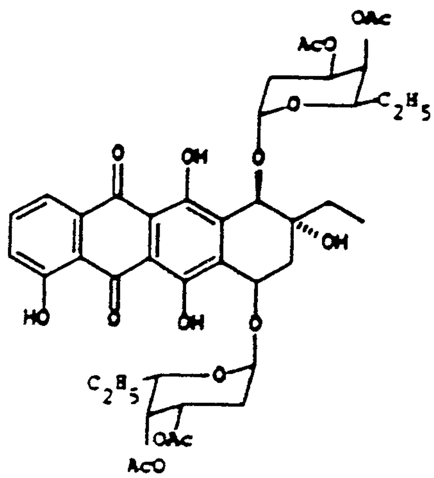

**(22b)**

13°) Anthracycline selon la revendication 9, caractérisée en ce qu'elle est constituée par la O-(di-O-acétyl-3′,4′ tridésoxy-2′,6′,7′-α-L-thréo-heptopyrannosyl)-7 β-Rhodomycinone de Formule 21b, ci-après :

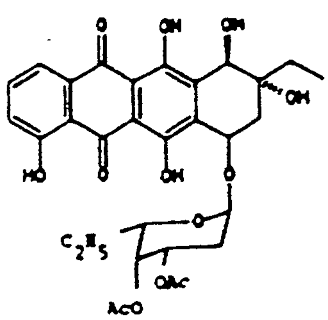

**(21b)**

14°) Anthracycline selon la revendication 9, caractérisée en ce qu'elle est constituée par la di-O-(di-O-acétyl-3′,4′ hexadésoxy-2′,6′,7′,8′,9′,10′-α-L-thréo décapyrannosyl)-7,10 β-Rhodomycinone de Formule 22c, ci-après :

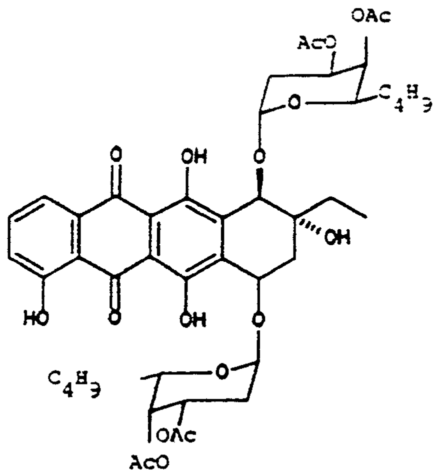

(22c)

15°) Anthracycline selon la revendication 9, caractérisée en ce qu'elle est constituée par la O-(di-O-acétyl-3',4' hexadésoxy-2',6',7',8',9',10'-α-L-thréodécapyrannosyl)-7 β-Rhodomycinone de Formule 21c, ci-après :

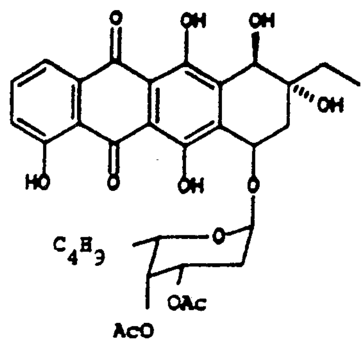

(21c)

16°) Anthracycline selon la revendication 8, caractérisée en ce qu'elle est constituée par l'acétoxy-3 O-acétyl-4' désamino-3' méthyl-6'daunorubicine de Formule 20b, ci-après :

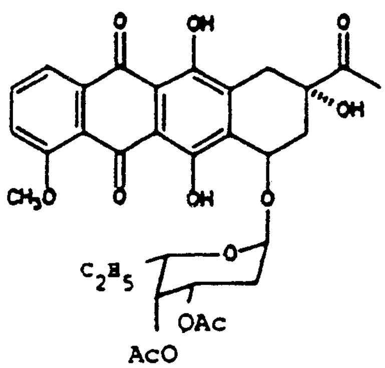

(20b)

17°) Anthracycline selon la revendication 8, caractérisée en ce qu'elle est constituée par l'acétoxy-3' O-acétyl-4' désamino-3' propyl-6' daunorubicine de Formule 20c, ci-après :

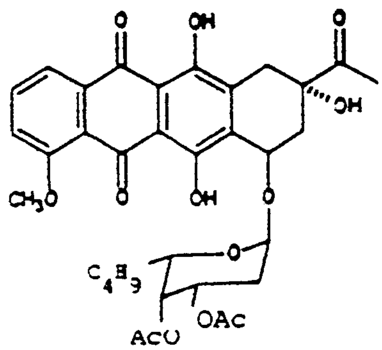

(20c)

18°) Di-O-isopropylidène-1,2,4,5 hexahydroxy-1, 2R, 3R, 4R, 5R, 6S heptane de Formule 8, ci-après :

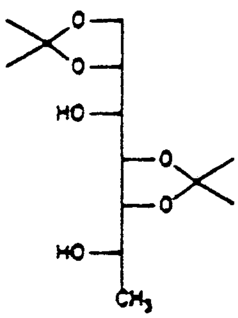

(8),

produit intermédiaire du procédé selon la revendication 2.

19°) Di-O-benzyl-3,6 di-O-isopropylidène-1,2,4-5 hexahydroxy-1, 2R, 3R, 4R, 6S heptane de Formule 9, ci-après :

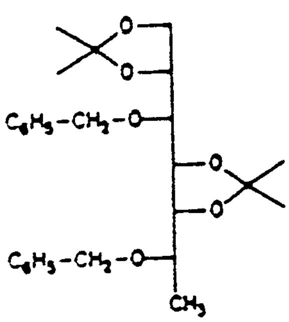

(9),

produit intermédiaire du procédé selon la revendication 2.

20°) Di-O-benzyl-3,6-hexahydroxy-1, 2R, 3R, 4R, 5R, 6S heptane de Formule 10, ci-après :

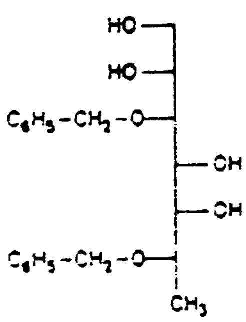

(10),

produit intermédiaire du procédé selon la revendication 2.

21°) Di-O-benzyl-2,5-α-L-fucofurannose de Formule 11, ci-après :

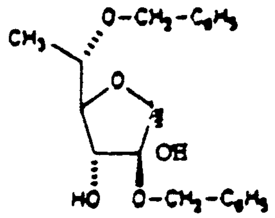

(11)

produit intermédiaire selon la revendication 2.

22°) Di-O-benzyl-2,5-β-L-fucofurannose de Formule 11, ci-après :

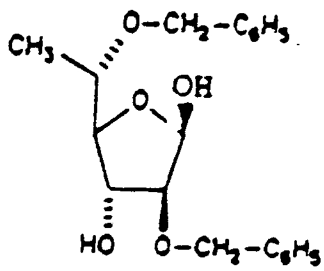

(11)

produit intermédiaire selon la revendication 2.

23°) Di-O-benzoyl-3,6 di-O-isopropylidène-1, 2, 4, 5 hexahydroxy 1,2R,3R,4R,5R,6R octane de Formule 15b, ci-après :

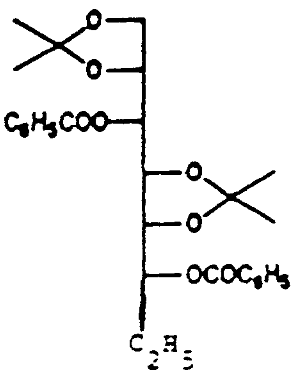

(15b),

produit intermédiaire pour la préparation des analogues du L-Fucose, selon la revendication 1

24°) Di-O-benzoyl-3,6 di-O-isopropylidène-1,2,4,5 hexahydroxy- 1,2R,3R,4R,5R,6S Octane de Formule 14b, ci-après :

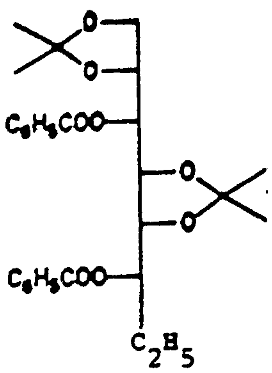

(14b),

produit intermédiaire pour la préparation des analogues du L-Fucose, selon la revendication 1.

25°) Di-O-benzoyl-3,6 di-O-isopropylidène-1,2,4,5 hexahydroxy-1,2R,3R,4R,5R,6S décane de Formule 15c, ci-après :

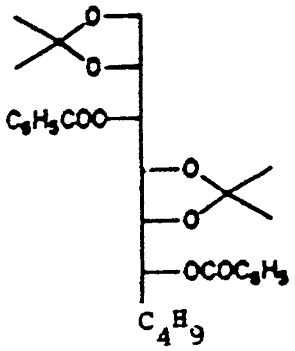

(15c),

produit intermédiaire pour la préparation des analogues du L-Fucose, selon la revendication 1

26°) Di-O-benzoyl-3,6 di-O-isopropylidene-1,2,4,5 hexahydroxy-1,2R,3R,4R,5R, 6S décane de Formule 14c, ci-après :

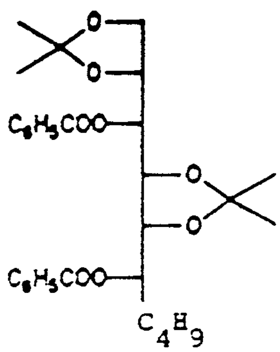

(14c),

produit intermédiaire pour la préparation des analogues du L-Fucose, selon la revendication 1.

27°) Di-O-benzoyl-3,6 hexahydroxy-1, 2R, 3R, 4R, 5R, 6S octane de Formule 16b, ci-après :

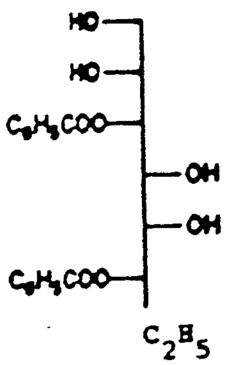

(16b),

produit intermédiaire pour la préparation des analogues du L-Fucose, selon la revendication 1.

28°) Di-O-benzoyl-2,5 méthyl-6 α (et β)-L-Fucofurannose de Formule 17b, ci-après :

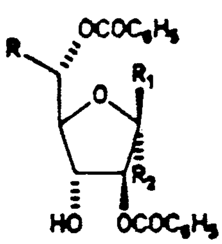

(17b),

produit intermédiaire pour la préparation des analogues du L-Fucose, selon la revendication 1.

29°) Intermédiaire pour la préparation des glycals selon la revendication 3, de Formule 18 :

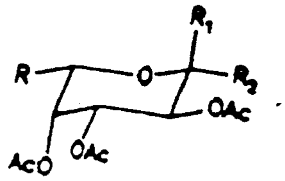

(18)

dans laquelle :
**R** représente un groupe alkyle éventuellement substitué, linéaire ou ramifié ou un groupe aryle éventuellement substitué, à l'exception d'un groupe $CH_2OH$ ou d'un groupe $CH_2OR_3$, $R_3$ ayant la même signification que R ;
**$R_1$ et $R_2$** sont identiques ou différents et représentent un groupe acétoxy ou un atome d'hydrogène ;

30°) Composition pharmaceutique,caractérisée en ce qu'elle contient en tant que composé actif une anthracycline selon les revendications 8 à 16, éventuellement asso cline selon les revendications 8 à 16, éventuellement associée à au moins un véhicule pharmaceutiquement acceptable.